(19) Europäisches Patentamt
European Patent Office
Office européen des brevets

(11) **EP 2 297 321 B1**

(12) **EUROPÄISCHE PATENTSCHRIFT**

(45) Veröffentlichungstag und Bekanntmachung des Hinweises auf die Patenterteilung:
**09.01.2013 Patentblatt 2013/02**

(21) Anmeldenummer: **09757275.4**

(22) Anmeldetag: **03.06.2009**

(51) Int Cl.:
**C12N 15/10** *(2006.01)*    **C12Q 1/68** *(2006.01)*

(86) Internationale Anmeldenummer:
**PCT/EP2009/003945**

(87) Internationale Veröffentlichungsnummer:
**WO 2009/146892 (10.12.2009 Gazette 2009/50)**

(54) **VERFAHREN ZUR ERZEUGUNG EINER VARIANTEN-BIBLIOTHEK VON DNA-SEQUENZEN**

PROCESS FOR GENERATING A VARIANT LIBRARY OF DNA SEQUENCES

PROCÉDÉ DE PRODUCTION D'UNE BIBLIOTHÈQUE DE VARIANTS DE SÉQUENCES D'ADN

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **05.06.2008 EP 08010256**

(43) Veröffentlichungstag der Anmeldung:
**23.03.2011 Patentblatt 2011/12**

(73) Patentinhaber: **c-LEcta GmbH**
**04103 Leipzig (DE)**

(72) Erfinder:
• **GREINER-STÖFFELE, Thomas**
**04279 Leipzig (DE)**
• **FELLER, Claudia**
**04103 Leipzig (DE)**
• **STRUHALLA, Marc**
**04229 Leipzig (DE)**

(74) Vertreter: **Bülle, Jan et al**
**Patentanwälte Kutzenberger & Wolff**
**Theodor-Heuss-Ring 23**
**50668 Köln (DE)**

(56) Entgegenhaltungen:
EP-A- 0 466 083    WO-A-01/12802
WO-A-02/34762    WO-A-97/46670
WO-A-98/32845    WO-A-98/58080
WO-A-2006/047669    WO-A1-03/106679

• OSUNA J ET AL: "COMBINATORIAL MUTAGENESIS OF THREE MAJOR GROOVE-CONTACTING RESIDUES OF ECORI SINGLE AND DOUBLE AMINO ACID REPLACEMENTS RETAINING METHYLTRANSFERASE-SENSITIVE ACTIVITIES" GENE, ELSEVIER, AMSTERDAM, NL, Bd. 106, Nr. 1, 1. Januar 1991 (1991-01-01), Seiten 7-12, XP002216327 ISSN: 0378-1119
• GE L ET AL: "SIMULTANEOUS INTRODUCTION OF MULTIPLE MUTATIONS USING OVERLAP EXTENSION PCR" BIOTECHNIQUES, INFORMA LIFE SCIENCES PUBLISHING, WESTBOROUGH, MA, US, Bd. 22, Nr. 1, 1. Januar 1997 (1997-01-01), Seite 28,30, XP000676361 ISSN: 0736-6205 in der Anmeldung erwähnt
• WEISBERG E P ET AL: "SIMULTANEOUS MUTAGENESIS OF MULTIPLE SITES: APPLICATION OF THE LIGASE CHAIN REACTION USING PCR PRODUCTS INSTEAD OF OLIGONUCLEOTIDES" BIOTECHNIQUES, INFORMA LIFE SCIENCES PUBLISHING, WESTBOROUGH, MA, US, Bd. 15, Nr. 1, 1. Juli 1993 (1993-07-01), Seiten 68-70,72, XP000385832 ISSN: 0736-6205 in der Anmeldung erwähnt
• WATARU ITO ET AL: "A GENERAL METHOD FOR INTRODUCING A SERIES OF MUTATIONS INTO CLONED DNA USING THE POLYMERASE CHAIN REACTION" GENE, ELSEVIER, AMSTERDAM, NL, Bd. 102, Nr. 1, 1. Januar 1991 (1991-01-01), Seiten 67-70, XP000303855 ISSN: 0378-1119
• SHAYIQ R M ET AL: "MULTIPLE IN VITRO SITE-DIRECTED MUTAGENESIS USING ASYMMETRIC POLYMERASE CHAIN REACTION" ANALYTICAL BIOCHEMISTRY, ACADEMIC PRESS INC. NEW YORK, Bd. 221, Nr. 1, 15. August 1994 (1994-08-15), Seiten 206-208, XP000457278 ISSN: 0003-2697 in der Anmeldung erwähnt

- LING M M ET AL: "APPROACHES TO DNA MUTAGENESIS: AN OVERVIEW" ANALYTICAL BIOCHEMISTRY, ACADEMIC PRESS INC. NEW YORK, Bd. 254, Nr. AB972428, 1. Januar 1997 (1997-01-01), Seiten 157-178, XP000955621 ISSN: 0003-2697

- HORTON R M ET AL: "Engineering hybrid genes without the use of restriction enzymes: gene splicing by overlap extension" GENE, ELSEVIER, AMSTERDAM, NL, Bd. 77, 1. Januar 1989 (1989-01-01), Seiten 61-68, XP002090392 ISSN: 0378-1119

**Beschreibung**

Die Erfindung betrifft ein Verfahren zur Erzeugung einer Varianten-Bibliothek von DNA-Sequenzen.

Im Stand der Technik sind Verfahren zur Erzeugung einzelner bestimmter Varianten einer DNA-Sequenz beschrieben, wobei zum Beispiel verwiesen werden kann auf L. Ge et al., Biotechniques, 22 (1), 1997, 28-30; E. P. Weisberg et al., Biotechniques, 15 (1). 1993, 68-76; R. M. Shayiq et al., Analytical Biochemistry, 221 (1), 1994, 206-208; oder W. Ito et al., Gene, 102 (1), 1991, 67-70. Bei diesen Verfahren werden in eine DNA-Sequenzgleichzeitig mehrere Mutationen eingeführt. Mit PCR werden einzelne Fragmente einer DNA-Sequenz erzeugt und wieder verbunden. Über Mismatch-Primer werden hierbei Mutationen eingeführt, wobei an jede Mutationsstelle lediglich ein Primer bindet. Hierbei geht es jedoch nicht um die Erzeugung einer Varianten-Bibliothek, sondern um die Erstellung einer bestimmten einzelnen Variante einer DNA-Sequenz.

Verfahren zur Erzeugung einer Varianten-Bibliothek von DNA-Sequenzen sind ebenfalls im Stand der Technik bekannt In diesem Zusammenhang kann beispielsweise verwiesen werden auf WO 01112802, WO 02/34762, Wong T. S. et al., The Diversity Challenge in Directed Protein Revolution, Combinatorial Chemistry & High Throuput Screening, 9 (4), 2006, 271-288; und C. Neylon, Nucleic Acids Research, 2004, 32(4), 1448-59.

WO 01/12802 und WO 02/34762 offenbaren Verfahren zur Erzeugung einer Varianten-Bibliothek von DNA-Sequenzen. Die Verfahren nutzen Oligonukleotid-Gemische und eine bestimmte Art von PCR, wobei an jede Mutationsstelle lediglich ein Primer bindet Eine Unterteilung der Ausgangssequenz in mehrere Sequenzabschnitte und deren Amplifikation erfolgt nicht.

WO 03/106679 beschreibt ein Verfahren, um die Redundanz in randomisierten Genom-Bibliotheken zu entfernen.

J Osuna et al. (Gene, 106(1), 1991, 7-12) umfasst eine Studie zur Mutagenese des Restriktionsenzymes EcoRI und beschreibt ein Verfahren zur Erzeugung einer Varianten-Bibliothek von DNA-Sequenzen.

Ein weiteres aus dem Stand der Technik bekanntes Verfahren ist das sog. "DNA-Shuffling" (vgl. z.B, WO 98/32845, WO 98/58080. WO 2006/047669). Dabei werden räumlich getrennte Abschnitte miteinander rekombiniert (*geshuffelt*). Es werden keine Mutationen durch Oligonukleotide eingeführt und die eingesetzten Oligonukleotide binden auch nicht an Mutationsstellen.

Proteine finden vielfältige Anwendungen in Forschung und Industrie. Viele technische Anwendungen für Proteine machen es erforderlich, die Eigenschaften von natürlichen Proteinen den besonderen Anforderungen der jeweiligen technischen Anwendung anzupassen. Hierfür führt man künstliche Veränderungen in die Proteine ein, welche die gewünschte Verbesserung einer Eigenschaft des Proteins herbeiführen. Dieses Vorgehen nennt man *"Protein Engineering"*.

Die Strukturaufklärung nativer Proteine hat in den letzten Jahren dazu geführt, dass für eine sehr große Zahl Proteinen detaillierte Kenntnisse über Sequenz, Struktur und über Struktur-Wirkungs-Beziehungen vorliegen. Dennoch sind Versuche, die Eigenschaften von Proteinen durch rationales Protein Design zu verändern, häufig nicht erfolgreich. Oft werden statistische Methoden angewendet, bei denen mit Hilfe von Mutagenese-Verfahren eine Varianten-Bibliothek erzeugt wird, welche eine große Anzahl von Protein-Varianten umfasst , die dann im Hinblick auf Protein-Varianten mit verbesserten Eigenschaften untersucht wird.

Infolge der detaillierten Kenntnisse über die Struktur der nativen Proteine erscheint es vielfach sinnvoll, die Wildtyp-Sequenz keiner völlig willkürlichen Mutagenese zu unterziehen, sondern die Mutationen auf bestimmte Aminosäure-Positionen des Proteins zu beschränken (fokussierte Mutagenese). Dadurch lässt sich die theoretisch mögliche Komplexität der Bibliothek einschränken. So lassen sich zum Beispiel in Proteinen Aminosäure-Positionen identifizieren, die für ein bestimmtes Bindevermögen oder im Falle von Enzymen für die Substraterkennung zuständig sind. Aber auch für eher globale Eigenschaften wie die Protein-Stabilität lassen sich besonders relevante Aminosäure-Positionen identifizieren.

Ebenfalls ist es wünschenswert, die an einzelnen Aminosäure-Positionen zugelassenen Mutationen auf bestimmte Aminosäure-Austausche zu beschränken. Zum Beispiel erlaubt die gesuchte Zieleigenschaft des veränderten Proteins die sinnvolle Einschränkung der zugelassenen Aminosäuren. Eine sehr plausible Einschränkung der zugelassenen Aminosäure-Positionen an einzelnen Positionen lässt sich ebenfalls aus den umfangreichen Sequenzdaten in Sequenz-Datenbanken ableiten. Über Sequenz-Alignments können für einzelne Aminosäure-Positionen die Aminosäuren identifiziert werden, die natürlich vorkommende Proteine an dieser Stelle aufweisen. Entsprechend können dann die Aminosäure-Austausche an diesen Positionen in der Mutagenese auf die natürlich vorkommenden Aminosäuren eingeschränkt werden.

Bei der Erzeugung von fokussierten Varianten ist es also wünschenswert, ein möglichst hohes Maß an Kontrolle zu erreichen, nicht nur im Hinblick auf die relative Lage der Veränderungen innerhalb der Gesamtsequenz, sondern auch im Hinblick auf die Anzahl der Veränderungen pro Gesamtsequenz und die Arten der Veränderungen.

Im Stand der Technik sind eine Reihe von Mutagenese-Verfahren bekannt. Eine einfache Möglichkeit besteht in der Durchführung einer fehlerhaften PCR (*Error-Prone* PCR), bei der eine Polymerase bei der DNA-Amplifikation fehlerhaft Nukleotide in die DNA-Sequenz einbaut und dadurch Mutationen erzeugt. Eine weitere Möglichkeit zu Einführung von Mutationen in DNA-Sequenzen stellt das *DNA-Shuffling* dar.

Es sind auch Mutagenese-Verfahren beschrieben, die mit Hilfe von Oligonukleotiden funktionieren. In diesem Zusammenhang kann beispielsweise auf WO 02/34762 und WO 01/12802 verwiesen werden. Generell können mit derartigen Mutagenese-Verfahren mit Hilfe von Oligonukleotiden über Mismatch-Positionen Mutationen in DNA-Sequenzen eingeführt werden. Somit lassen sich zum Beispiel mit Hilfe eines randomisierten Oligonukleotids an einer Aminosäure-Position alle 20 natürlichen Aminosäuren einsetzen (*saturation mutagenesis*). Eine besondere Ausführungsform dieser Art von Mutagenese ist die Quickchange-Mutagenese, die auch als *Multiple-Quickchange-Mutagenese* ausgeführt werden kann. Ein ähnliches Verfahren beschreibt die Massive *Mutagenesis.* Bei beiden Verfahren werden mehrere bis viele Oligonukleotide bei der Polymerase-vermittelten Amplifikation einer zu mutierenden DNA-Sequenz eingesetzt. Ein Nachteil beider Verfahren liegt in der nicht möglichen Steuerung, wie viele Oligonukleotide pro Amplifikat verwendet werden. Einzelne Mutationsstellen können durch die Polymerase überlesen werden. Dadurch unterliegt die Anzahl der Mutationen pro Variante starken Schwankungen und kann nicht kontrolliert werden. Die theoretische Gesamt-Komplexität der Bibliothek kann dadurch nicht ausreichend eingeschränkt werden. Ein weiterer Nachteil tritt auf, wenn mehrere Oligonukleotide an eine Mutationsstelle binden können. Unterschiede, die durch eine Varianz der Affinität der Oligonukleotide zu der Mutationsstelle verursacht werden, können nicht ausgeglichen werden. Ein weiterer Nachteil der Verfahren besteht in der Tatsache, dass die durchschnittliche Anzahl der Mutationen pro Variante mit einer steigenden Anzahl von Mutationsstellen zunehmend kleiner ist als die Anzahl der Mutationsstellen. Dadurch lassen sich Bibliotheken, bei denen mehrere Mutationen kombiniert werden sollen, nur in sehr großen Komplexitäten erzeugen, in denen der Anteil der Varianten, welche die gewünschte Kombination von mehreren Mutationen aufweisen, nur eine kleine Fraktion der Bibliothek ausmacht.

Es besteht ein Bedarf an Verfahren zur Erzeugung einer Varianten-Bibliothek von DNA-Sequenzen,

- bei denen die Mutationen auf mehrere, genau determinierte Positionen der DNA-Sequenz (bzw. Protein-Sequenz) beschränkt werden können,
- bei denen die Mutationsstellen über die gesamte DNA-Sequenz verteilt werden können,
- bei denen festgelegt werden kann, welche Mutationen an welcher Position zugelassen sind,
- die einfach und mit Standard-Labormethoden durchzuführen sind,
- bei denen die Verteilung einzelner Mutationen in der Bibliothek sehr genau gesteuert werden kann,
- bei denen auch benachbarte Mutationen kontrolliert eingeführt werden können,
- bei denen die Gesamtanzahl der Mutationen pro Variante genau gesteuert werden kann und
- bei denen die Komplexität der erzeugten Bibliothek präzise eingeschränkt werden kann.

Insbesondere besteht ein Bedarf an Verfahren, mit denen Varianten-Bibliotheken mit einem hohen Anteil an Varianten, in denen mehrere Mutationen miteinander kombiniert sind, erzeugt werden können.

Der Erfindung liegt die Aufgabe zugrunde, ein Verfahren zur Erzeugung einer Varianten-Bibliothek von DNA-Sequenzen bereitzustellen, welches Vorteile gegenüber den Verfahren des Standes der Technik aufweist, insbesondere die vorstehend genannten Vorteile.

Diese Aufgabe wird durch den Gegenstand der Patentansprüche gelöst.

Die Erfindung betrifft ein Verfahren zur Erzeugung einer Varianten-Bibliothek von DNA-Sequenzen ausgehend von mindestens einer DNA-Ausgangssequenz umfassend die folgenden Schritte:

a) Auswählen von mindestens zwei Mutationsstellen in der DNA-Ausgangssequenz;

b) Unterteilen der DNA-Ausgangssequenz in mindestens zwei Sequenzabschnitte in einer Weise, so dass mindestens zwei dieser Sequenzabschnitte jeweils mindestens eine der Mutationsstellen umfassen;

c) Amplifizieren der Sequenzabschnitte durch PCR mit Hilfe von insgesamt wenigstens fünf verschiedenen Oligonukleotiden, wobei

(i) sich an jeder Mutationsstelle mindestens eines der Oligonukleotide anlagern kann;

(ii) sich an mindestens eine Mutationsstelle wenigstens zwei der Oligonukleotide anlagern können; und

(iii) in den durch PCR erhaltenen doppelsträngigen Amplifikations-Produkten (Fragmenten) durch die Oligonukleotide an den Mutationsstellen über *Mismatch*-Positionen Mutationen eingeführt werden, wobei an mindestens einer Mutationsstelle mindestens zwei Mutationen eingeführt werden: und

d) Verknüpfen der doppelsträngigen Amplifikations-Produkte zu DNA-Sequenzen, wobei

(i) für mindestens 75% der Varianten in der Varianten-Bibliothek die Anzahl der Mutationen pro Variante im Bereich N liegt, wobei $X \geq N \geq (X-3)$ mit $X \in N$ und $25 > X > 3$, oder

(ii) die größte Fraktion der Varianten eine Anzahl d von Mutationen aufweist und die Fraktion der Varianten, welche d+2 Mutationen aufweist, höchstens 10% der Gesamtzahl der Varianten in der Varianten-Bibliothek ausmacht.

Bei dem erfindungsgemäßen Verfahren handelt es sich bevorzugt um ein Mutagenese-Verfahren, nicht jedoch um ein Rekombinations-Verfahren. Im Sinne der Erfindung wird unter einem Mutagenese-Verfahren die Erzeugung einer großen Anzahl von Varianten bevorzugt ausgehend von einer DNA-Ausgangssequenz verstanden. Mutagenese-Verfahren sind abzugrenzen von Rekombinations-Verfahren, bei denen eine Anzahl von Varianten einer DNA-Sequenz miteinander kombiniert wird.

Im Sinne des erfindungsgemäßen Verfahrens bedeutet "anlagern", dass infolge der Basenpaarung zwischen Sequenzabschnitt und Oligonukleotid ein hinreichend stabiler Komplex entsteht, welcher als Startpunkt einer PCR dienen kann. Üblicherweise bindet dazu das Oligonukleotid über mehrere Nukleobasen an den Sequenzabschnitt, wobei die Mutationsstelle auf beiden Seiten vom gebildeten Doppelstrang flankiert wird. Es ist nicht erforderlich, dass dabei alle Nukleotide des Oligonukleotids an der Bindung beteiligt sind. Ggf. vorhandene *Mismatch*-Positionen führen dann im Zuge der PCR ortsgerichtete Mutationen ein. Darüber hinaus können über sogenannte 5'-Überhänge Sequenzverlängerungen durch die Oligonukleotide angehängt werden, die eine anschließendes Verknüpfen der Sequenzabschnitte ermöglichen.

Das erfindungsgemäße Verfahren eignet sich zur Herstellung einer Varianten-Bibliothek von DNA-Sequenzen. Diese DNA-Sequenzen können dann in eine Varianten-Bibliothek aus Protein-Molekülen übersetzt werden, bei denen an definierten Aminosäure-Positionen gezielte Mutationen eingeführt werden.

Das erfindungsgemäße Verfahren wird bevorzugt gezielt durchgeführt, d.h. verläuft gerichtet, rational, wohlüberlegt bzw. zu einem gewissen Grad vorherbestimmt ab, zumindest teilweise, nicht jedoch willkürlich oder zufällig (randomisiert). Dies betrifft bevorzugt insbesondere die Auswahl der mindestens zwei Mutationsstellen in der DNA-Ausgangssequenz gemäß Schritt (a), die (gedankliche) Unterteilung in zwei Sequenzabschnitte gemäß Schritt (b) und/ oder die Auswahl der wenigstens fünf verschiedenen Oligonukleotide in Schritt (c).

Darin unterscheidet sich das erfindungsgemäße Verfahren von solchen Verfahren des Standes der Technik, welche randomisiert ablaufen (z.B. mit Hilfe von E. coli XL1 red, UV Bestrahlung, chemische Mutagenese wie Deaminierung oder Alkylierung, DNA Shuffling, oder fehlerhafte PCR *(error prone)).*

Da die Mutationen bei dem erfindungsgemäßen Verfahren an den Mutationsstellen über Mismatch-Positionen eingeführt werden, basiert das erfindungsgemäße Verfahren auf dem Prinzip der ortsgerichteten Mutagenese *(site directed mutagenesis).* Darin unterscheidet sich das erfindungsgemäße Verfahren von solchen Verfahren des Standes der Technik, welche auf DNA-Shuffling oder auf einer *error prone* PCR basieren.

Im Stand der Technik ist ferner die sog. *site directed random mutagenesis* bekannt, bei der eine PCR mit Hilfe teilweise degenerierter Primer durchgeführt wird. Bei degenerierten Primern werden bei der Synthese der Primer Nukleotid-Gemische eingesetzt. Vorzugsweise kommen bei dem erfindungsgemäßen Verfahren jedoch keine derart degenerierten Primer (Oligonukleotide) zum Einsatz, d.h. ein randomisierter Sequenzabschnitt ist auf den Oligonukleotiden vorzugsweise nicht vorhanden.

Das Verfahren greift zurück auf etablierte Methoden der Molekularbiologie. Über Oligonukleotide können Punktmutationen durch PCR-Amplifikation eingeführt werden *(Site Directed Mutagenesis).* Die entstandenen Fragmente können durch PCR oder durch Restriktionsenzyme und Ligasen wieder miteinander verknüpft werden. In diesem Zusammenhang kann beispielsweise verwiesen werden auf S. Brakmann et al., Directed Molecular Evolution of Proteins, VCH-Wiley, 1st edition, 2002; F.H. Arnold, Directed Enzyme Evolution: Screening and Selection Methods, Humana Press, 1 st edition, 2003.

Mit dem erfindungsgemäßen Verfahren können aus mehreren Sequenzabschnitten, welche Mutationsstellen enthalten, separat Fragmente erzeugt werden, welche dann an den jeweiligen Mutationsstellen Mutationen aufweisen.

Ferner können mit dem erfindungsgemäßen Verfahren ausgehend von einem einzelnen Sequenzabschnitt, der wenigstens eine Mutationsstelle enthält, parallel mehrere verschiedene Fragmente erzeugt werden, welche sich durch die Art der jeweiligen Mutation an dieser Mutationsstelle unterscheiden. Dies wird dadurch erreicht, dass verschiedene Oligonukleotide, welche bei der Amplifikation durch PCR als Primer fungieren, an dieselbe Mutationsstelle anlagern können, dabei jedoch durch verschiedene Mismatch-Positionen verschiedene Mutationen erzeugen. Indem die PCR-Fragmente parallel erzeugt werden, können die Fragmente nach Erzeugung quantifiziert und dann in einem exakt kontrollierten Verhältnis gemischt werden. Dadurch werden ungewollte Ungleichgewichte in der Mutations-Verteilung an der entsprechenden Mutationsstelle vermieden. Enthält das DNA-Fragment nur eine Mutationsstelle und sollen an dieser Mutationsstelle zum Beispiel 20 Mutationen eingeführt werden, so sind 20 PCR-Reaktionen parallel durchzuführen. Befinden sich auf dem Fragment zwei Mutationsstellen, so sind alle möglichen Kombinationen der Mutationen an beiden Mutationsstellen parallel durchzuführen. Natürlich können die Oligonukleotide auch als Gemisch in den PCR-Reaktionen eingesetzt werden, wobei dadurch mit Ungleichgewichten in der Mutationsverteilung gerechnet werden muss. Um eine zu hohe Anzahl von PCR-Fragmenten zu vermeiden, kann auch die nächstfolgende Mutationsstelle in der DNA-Sequenz auf das nächste DNA-Fragment verlegt werden. Werden keine Oligonukleotid-Gemische eingesetzt, ist es in der Regel trotzdem möglich, mit einigen Dutzend parallel durchgeführten PCR-Reaktionen eine Varianten-Bibliothek mit Komplexitäten von mehr als 100.000 Varianten bis hin zu Bibliotheken mit mehr als 1 Million Varianten zu erzeugen.

In einer bevorzugten Ausführungsform wird das erfindungsgemäßen Verfahren angewendet, um eine DNA-Bibliothek kodierend für eine Bibliothek von Protein-Varianten mit veränderten Bindungseigenschaften, bzw. im Falle eines Enzyms, mit veränderter Substrat-Spezifität zu erzeugen. Die Mutationsstellen werden dabei beschränkt auf Aminosäure-Positionen, die abgeleitet aus Strukturdaten oder Struktur-Modellen für die Ligand-Erkennung im Falle eines bindenden Proteins (z. B. Antikörper) oder die Substrat-E kennung im Falle eines Enzyms verantwortlich sind. Zum Beispiel kann die Mutagenese fokussiert werden auf solche Aminosäure-Positionen in der Substrat-Bindungstasche eines Enzyms, die für die Substratbindung verantwortlich sein können und die keine katalytischen Aminosäure-Reste sind.

In einer weiteren bevorzugten Ausführungsform werden an den ausgewählten Mutationsstellen nur solche Aminosäuren zugelassen, die auch in natürlichen Protein-Sequenzen vorkommen. Diese können über Sequenz-Vergleiche mit Sequenz-Datenbanken identifiziert werden. Über Homologie-Vergleiche der Ausgangs-Sequenz können in Sequenz-Alignments für einzelne Aminosäure-Positionen diejenigen Aminosäuren identifiziert werden, die in anderen Proteinen an dieser Stelle vorkommen. Dies ist ein Verfahren, mit der in der zu erzeugenden Varianten-Bibliothek die Diversität der Natur in den ausgewählten Positionen abgebildet werden kann.

Es wurde überraschend gefunden, dass sich mit dem erfindungsgemäßen Verfahren die Mutationsverteilungen der resultierenden Varianten-Bibliotheken sehr genau steuern lassen. Zum Beispiel ist es möglich, dass alle Varianten der Varianten-Bibliothek die gleiche Anzahl an Mutationen aufweisen. Das Vorkommen von unmutierten Sequenzen (Wildtyp) kann ausgeschlossen werden, bzw. sehr genau gesteuert werden. Wenn man zum Beispiel 6 Mutationen pro Protein einführen möchte, so ist es einfach möglich, dass in der Tat der größte Teil der Varianten-Bibliothek auch 6 Mutationen aufweist. Es sind keine Varianten mit 7 Mutationen enthalten. Die Varianz der Anzahl der Mutationen pro Variante kann sehr genau gesteuert werden. Man kann also z.B. auch festlegen, wie viele Varianten in etwa 5 Mutationen haben sollen, und so weiter.

Die im Stand der Technik beschriebenen PCR-basierten Mutagenese-Verfahren zur Erzeugung von Varianten-Bibliotheken zeichnen sich dadurch aus, dass die Anzahl der Mutationen pro Variante in der Regel einer Normalverteilung unterliegt. Es gibt eine Fraktion von Varianten in der Bibliothek mit einer bestimmten Mutationsanzahl, die am häufigsten auftritt. Es sind in diesen Bibliotheken aber immer auch Varianten enthalten, die mehr Mutationen aufweisen und Varianten, die weniger Mutationen aufweisen. Die Mutations-verteilung kann nicht eingeschränkt werden und die Gesamt-Komplexität der Bibliotheken kann nicht ausreichend eingegrenzt werden. Wird eine Varianten-Bibliothek zum Beispiel an 5 Aminosäure-Positionen komplett randomisiert, ergibt sich für diese Varianten eine Komplexität von $20^5 = 3,2$ Millionen Varianten. Bei 6 Aminosäure-Positionen ergeben sich bereits $20^6 = 64$ Millionen und bei 7 Aminosäure-Positionen sogar $20^7 = 1,28$ Milliarden Varianten. Erzeugt man mit den im Stand der Technik beschriebenen PCR-basierten Verfahren eine Varianten-Bibliothek, die hauptsächlich 5 Aminosäure-Mutationen aufweist, so entstehen auch Fraktionen mit 6 und mit 7 Aminosäure-Austauschen. Zusätzlich entstehen zahlreiche Varianten, die weniger als 5 Aminosäure-Austausche aufweisen. Üblicherweise macht die größte Fraktion im Bezug auf die Anzahl der Mutationen etwa 20 bis 40% der erzeugten Bibliothek aus. Ein großer Anteil der Bibliothek weist also nicht die gewünschte Anzahl von Mutationen auf. Darüber hinaus lässt sich die theoretische Gesamt-Komplexität der Bibliothek nicht ausreichend einschränken. Es ist zum Beispiel nicht wünschenswert, eine Bibliothek mit einer theoretischen Komplexität von mehr als 1 Milliarde Varianten zu erzeugen, wenn man anschließend nur wenige 100.000 davon analysieren kann!

Das erfindungsgemäße Verfahren erlaubt es, Varianten einer DNA-Sequenz zu erzeugen, bei denen die vorstehend beschriebene nachteilige Mutationsverteilung vermieden werden können. Das Verfahren erlaubt es, eine Varianten-Bibliothek zu erzeugen, bei der die Anzahl der Mutationen N pro Variante für jede Variante gleich ist. Hierbei gilt bevorzugt $2 \leq N \leq 20$, besonders bevorzugt $3 \leq N \leq 10$ und ganz besonders bevorzugt $4 \leq N \leq 8$.

Ebenfalls können Varianten-Bibliotheken erzeugt werden, bei denen keine Varianten in der Bibliothek enthalten sind, die mehr als 2 Mutationen, bevorzugter mehr als 1 Mutation mehr aufweist, als die Anzahl der Mutationen der größten Fraktion der Varianten. Beträgt die Anzahl der Mutationen der größten Fraktion beispielsweise 5, so sind bevorzugt keine Varianten in der Bibliothek enthalten, die mehr als 7 Mutationen, bevorzugter mehr als 6 Mutationen aufweisen.

Mit Hilfe des erfindungsgemäßen Verfahrens lassen sich Varianten-Bibliotheken erzeugen, bei denen die Mutationsstellen über die gesamte DNA-Sequenz verteilt sein können. Bevorzugt weisen mindestens zwei Mutationsstellen der Varianten-Bibliothek einen Abstand von bevorzugt mindestens 30 Nukleotiden, bevorzugter mindestens 100 Nukleotiden, noch bevorzugter mindestens 200 Nukleotiden, am bevorzugten mindestens 300 Nukleotiden und insbesondere mindestens 500 Nukleotiden auf. In einer besonders bevorzugten Ausführungsform liegen drei Mutationsstellen der Varianten-Bibliothek auf der DNA-Sequenz so verteilt, dass der Abstand zwischen der ersten und der zweiten, sowie der Abstand zwischen der zweiten und der dritten Mutationsstelle jeweils mindestens 30 Nukleotide, bevorzugter mindestens 50 Nukleotide, noch bevorzugter mindestens 100 Nukleotide, am bevorzugten mindestens 150 Nukleotide und insbesondere mindestens 200 Nukleotide beträgt.

Das erfindungsgemäße Verfahren erlaubt die Erstellung von Varianten-Bibliotheken, in denen die theoretische Gesamt-Komplexität ausreichend eingeschränkt werden kann, ohne dass dadurch die durchschnittliche Anzahl der Mutationen reduziert werden muss. Zum Beispiel ist es einfach möglich, eine Bibliothek mit einer theoretischen Komplexität von kleiner 100.000 herzustellen, in der trotzdem mehr als 75% der Varianten mehr als 5 Mutationen aufweisen.

Das erfindungsgemäße Verfahren verfolgt das Ziel der Erzeugung einer Varianten-Bibliothek von DNA-Sequenzen. Die Varianten-Bibliothek umfasst per Definition keine einzelne Variante, sondern eine Vielzahl unterschiedlicher, Varianten von DNA-Sequenzen. Insofern unterscheidet sich das erfindungsgemäße Verfahren von Verfahren des Standes der Technik, welche zum Ziel die Erzeugung einer einzelnen Spezies (Variante) haben. Ebenfalls unterscheidet sich das erfindungsgemäße Verfahren von Verfahren des Standes der Technik, welche zum Ziel die Rekombination von einzelnen Varianten von DNA-Sequenzen haben. Bevorzugt verfolgt das erfindungsgemäße Verfahren das Ziel, eine Varianten-Bibliothek mit mindestens $10^1$ verschiedenen DNA-Sequenzen zu erzeugen, bevorzugter mindestens $10^2$, noch bevorzugter mindestens $10^3$, am bevorzugtesten mindestens $10^4$ und insbesondere mindestens $10^5$ verschiedenen DNA-Sequenzen.

Im Sinne der Erfindung ist eine Mutationsstelle bevorzugt eine Teilsequenz der Ausgangs-sequenz, an die im Zuge des erfindungsgemäßen Verfahren eines der bei der PCR-Amplifikation eingesetzten Oligonukleotide bindet und die einer Sequenz-Veränderung unterworfen wird. Eine Mutationsstelle kann im Zuge des erfindungsgemäßen Verfahrens mehrfach mutiert werden, d.h. mehrere Mutationen umfassen. Die Mutationen können innerhalb einer Mutationsstelle an mehreren Positionen eingeführt werden. Die Sequenz-Positionen, die verändert werden, müssen nicht notwendigerweise innerhalb der Binde-Sequenz eines Oligonukleotids liegen, werden dieser aber in der Regel zumindest direkt benachbart sein. Die veränderte Sequenz ist in jedem Fall Teil der Mutationsstelle. Es ist möglich, dass sich die Sequenzen von zwei Mutationsstellen im Bezug auf die Binde-Sequenz der Oligonukleotide überlappen. Werden im Zuge des erfindungsgemäßen Verfahrens mehrere zueinander in Konkurrenz tretende Oligonukleotide eingesetzt, die an dem gleichen Mutationsort wirken, so werden diese Oligonukleotid-Gruppen einer Mutationsstelle zugeordnet.

Im Sinne der Erfindung ist eine Mutation bevorzugt eine Veränderung der DNA-Sequenz an einer bestimmten Position. Die Mutation bezieht sich bevorzugt auf die DNA-Ebene, d.h. bevorzugt nicht auf die Protein-Ebene, für welche diese DNA ggf. kodiert. Eine Mutation kann dabei bestehen

- im Austausch eines Nukleotids oder im Austausch von zwei oder drei unmittelbar aufeinander folgender Nukleotide in der DNA-Ausgangssequenz (Substitution);

- in der Eliminierung eines Nukleotids; oder in der Eliminierung mehrerer, vorzugsweise von drei, sechs oder neun, unmittelbar aufeinander folgender Nukleotide in der DNA-Ausgangssequenz (Deletion); und/oder

- in der Einfügung eines Nukleotids; oder in der Einfügung mehrerer, vorzugsweise von drei, sechs oder neun, unmittelbar aufeinander folgender Nukleotide in der DNA-Ausgangssequenz (Insertion).

Substitution, Insertion und Deletion sind dem Fachmann bekannt. Diese Mutationen lassen sich durch Vergleich der DNA-Ausgangssequenz mit der Sequenz des Amplifikations-Produktes eindeutig identifizieren, wobei die nicht mutierte Ausgangssequenz als Bezug dient.

Unter einer Mutations-Position im Sinne des erfindungsgemäßen Verfahrens werden die Sequenz-Bereiche verstanden, an denen eine bestimmte Mutation wirksam wird. Im Falle von Substitutionen können die Mutations-Positionen durch die Benennung der betroffenen Nukleotid-Positionen in der Ausgangssequenz spezifiziert werden. Im Falle von Insertionen können die Mutations-Positionen durch die Nennung der beiden Nukleotid-Positionen spezifiziert werden, zwischen denen die Insertion liegt. Im Falle von Deletionen können die Mutations-Positionen durch die Nennung der deletierten Nukleotid-Positionen spezifiziert werden.

Eine einzelne Substitutions-Mutation betrifft einzelne Nukleotide oder bis zu drei innerhalb der Sequenz unmittelbar aufeinander folgende Nukleotide in der DNA-Ausgangssequenz; nicht jedoch mehrere Nukleotide, die innerhalb der Sequenz durch mindestens 1 Nukleotid, welches seinerseits keiner Mutation unterworfen wird, getrennt sind. Werden zwei Nukleotide einer Mutation unterworfen, die lediglich durch ein einziges Nukleotid voneinander getrennt sind, und gehören beide Nukleotide zu einem für eine Aminosäure-kodierenden Triplett, dann werden beide Nukleotide bevorzugt als eine einzige Mutation aufgefasst. Die Bestimmung der Zugehörigkeit von zwei Nukleotiden, welche lediglich durch ein einziges Nukleotid voneinander getrennt sind, zu einem für eine Aminosäure-kodierenden Triplett ist dem Fachmann bekannt und orientiert sich üblicherweise am Leseraster, welches durch das Startcodon einer Gen-Sequenz festgelegt ist.

Werden an einer Mutations-Position der DNA-Sequenz mehrere Substitutionen, Deletionen oder Insertionen eingeführt, so werden die entsprechenden Mutationen zu einer Gruppe von Mutationen zusammengefasst, die an der gleichen Position wirksam werden.

Eine Varianten-Fraktion im Sinne der vorliegenden Erfindung wird definiert über die Anzahl der Mutationen der jeweiligen Varianten. Alle Varianten mit einer bestimmten Anzahl von Mutationen werden in einer Fraktion zusammengefasst. Varianten mit jeweils z.B. sechs Mutationen gehören somit zur selben Varianten-Fraktion. Die Größe einer Varianten-Fraktion wird gemessen an der absoluten Anzahl von unterschiedlichen Varianten, die zu dieser Fraktion gehören. Identische Spezies, d.h. Varianten mit identischen Sequenzen, erhöhen somit nicht die Größe der Varianten-Fraktion.

Bei der Bibliotheks-Komplexität wird im Sinne der vorliegenden Erfindung unterschieden zwischen der theoretischen und der physikalischen Komplexität. Die theoretische Komplexität entspricht der Summe aller möglichen Varianten, die bei der Durchführung der Mutagenese möglich sind. Die physikalische Komplexität entspricht der Anzahl der Varianten, die bei der Durchführung der Mutagenese tatsächlich erzeugt werden.

Mit Hilfe des erfindungsgemäßen Verfahrens kann die theoretische Komplexität der Varianten-Bibliothek sehr gut eingeschränkt werden, wodurch es möglich ist, Varianten-Bibliotheken mit großen physikalischen Komplexitäten zu erzeugen, wobei die theoretische Komplexität der Bank nicht sehr viel größer ist, als die physikalische. Die physikalische Komplexität von im Zuge des erfindungsgemäßen Verfahrens erzeugten Varianten-Bibliotheken ist bevorzugt größer 10.000, bevorzugter größer 50.000, noch bevorzugter größer 100.000, am bevorzugten größer 500.000 und insbesondere größer 1.000.000 ist, wobei die theoretische Komplexität bevorzugt weniger als 50-mal, bevorzugter weniger als 25-mal, noch bevorzugter weniger als 10-mal, noch bevorzugter weniger als 5-mal, am bevorzugten weniger als 3-mal und insbesondere weniger als 2-mal so groß wie die physikalische Komplexität der erzeugten Bibliothek ist. In einer bevorzugten Ausführungsform ist die theoretische Komplexität genauso groß wie die physikalische Komplexität der Bibliothek.

Im Falle von Verfahren zur ungerichteten Mutagenese einer DNA-Sequenz ist die theoretische Komplexität nur sehr schwer zu berechnen. Die erzeugten Bibliotheken sind charakterisiert durch die oben beschriebenen nachteiligen Mutationsverteilungen, wobei jede Varianten-Fraktion unterschiedliche theoretische Komplexitäten aufweist. Die Varianten-Fraktion mit 2 Mutationen einer Bibliothek eines Gens, welches 200 Aminosäure-Codons umfasst, weist eine theoretische Komplexität von ca. 65 Millionen Varianten auf, wenn jeweils alle möglichen 19 Aminosäure-Austausche möglich sind. Die Fraktion mit drei Mutationen hat hingegen bereits eine theoretische Komplexität von ca. 246 Milliarden Varianten.

Im Falle einer gerichteten Mutagenese entspricht im Sinne des erfindungsgemäßen Verfahrens die theoretische Komplexität der Anzahl der möglichen Kombinationen der zulässigen Mutationen in der Bibliothek.

In einer bevorzugten Ausführungsform beschränken sich die Mutationen in einer Mutationsstelle auf DNA-Ebene auf ein einzelnes Triplett, welches auf Protein-Ebene in der DNA-Ausgangssequenz für eine bestimmte Aminosäure codiert.

Oligonukleotide sind dem Fachmann bekannt. Im Sinne der Beschreibung sind Oligonukleotide bevorzugt Oligodesoxyribonukleotide der vier Desoxyribonukleotide Desoxyadenosin, Desoxyguanosin, Desoxycytidin und Desoxythymidin. Diese Oligonukleotide sind kommerziell erhältlich und lassen sich in grundsätzlich jeder beliebigen Sequenz mit Methoden herstellen, welche dem Fachmann bekannt sind, beispielsweise in Festphasensynthesen mit Hilfe geeigneter Syntheseroboter ausgehend von Phosphoramiditen. In diesem Zusammenhang kann beispielsweise vollumfänglich verwiesen werden auf C.W. Dieffenbach et al., PCR Primer: A Laboratory Manual, Cold Spring Harbor Laboratory Press, 2nd edition, 2003.

In einer bevorzugten Ausführungsform wird bei der Herstellung der Oligonukleotide in mindestens einer Position ein Nukleotid-Gemisch-eingesetzt, so dass diese Position randomisiert ist. Wird ein Gemisch aller vier Oligodesoxyribonukleotide bzw. der korrespondierenden Phopshoramidite eingesetzt, so werden bei einer randomisierten Position insgesamt vier verschiedene Oligonukleotide erzeugt, welche sich zumindest in dieser Position voneinander unterscheiden. Bei zwei jeweils vollständig randomisierten Positionen werden entsprechend insgesamt 16 verschiedene Oligonukleotide erzeugt. Bei der Amplifikation durch PCR konkurrieren diese Oligonukleotide dann um dieselbe Mutationsstelle und führen dort in Abhängigkeit davon, ob es sich um eine *Match*- oder eine *Mismatch*-Position handelt, eine Mutation ein. Wurde bei der Synthese der Oligonukleotide bei einer Position ein Gemisch aller vier Oligodesoxyribonukleotide bzw. der korrespondierenden Phopshoramidite eingesetzt, so ist ein resultierendes Oligonukleotid zwangsläufig ein *Match*-Oligonukleotid und die übrigen drei Oligonukleotide sind *Mismatch*-Oligonukleotide.

Im Sinne des erfindungsgemäßen Verfahrens wird unter einer "PCR" ein Verfahren zur Amplifikation einer DNA-Sequenz (die Ausgangs-Sequenz) verstanden, bei dem mit Hilfe von mindestens zwei Primern ein definierter Sequenz-Bereich mittels einer DNA-Polymerase durch zyklische Wiederholungen von Denaturierungs-, Annealing- und Elongations-Schritten vermehrt wird, wobei die Primer den amplifizierenden Sequenz-Bereich flankieren und sich an die entsprechenden Bereich der Ausgangs-Sequenz anlagern. Das Amplifizieren von DNA-Sequenzabschnitten durch PCR ist dem Fachmann bekannt, u.a. auch die *Overlap Extension PCR* (mitunter auch als *"Splicing by Overlap Extension PCR",* SOE-PCR bezeichnet) und *Outside Cutter.* In diesem Zusammenhang kann beispielsweise vollumfänglich verwiesen werden auf M.J. McPherson et al., PCR (The Basics), Taylor & Francis, 2nd edition, 2006; und M. Altshuler, PCR Troubleshooting: The Essential Guide, Caister Academic Press, 2006.

In Schritt a) des erfindungsgemäßen Verfahrens werden mindestens zwei Mutationsstellen in der DNA-Ausgangssequenz ausgewählt.

Die Gesamtzahl m der Mutationsstellen in der DNA-Ausgangssequenz ist grundsätzlich frei wählbar. Es gilt m $\geq 2$, $\geq 3$ oder $\geq 4$, bevorzugt m $\geq 5$, $\geq 6$ oder $\geq 7$, bevorzugter m $\geq 8$, $\geq 9$ oder $\geq 10$, noch bevorzugter m $\geq 11$, $\geq 12$ oder $\geq 13$, am bevorzugtesten m $\geq 14$, $\geq 15$ oder $\geq 16$.

Unter DNA-Ausgangssequenz versteht man im Sinne der Beschreibung vorzugsweise eine DNA-Sequenz, welche nativ sein kann (Wildtyp-Sequenz), jedoch nicht zwangsläufig nativ sein muss. Das erfindungsgemäße Verfahren kann auch von mehreren verschiedenen DNA-Ausgangssequenzen ausgehen. Bevorzugt geht das erfindungsgemäße Verfahren von einer einzelnen DNA-Ausgangssequenz aus.

Die Sequenzlänge der DNA-Ausgangssequenz ist prinzipiell beliebig. Bevorzugt umfasst die DNA-Ausgangssequenz 50 bis 10.000 Nukleotide, bevorzugter 100 bis 5.000 Nukleotide, noch bevorzugter 200 bis 3.000 Nukleotide, am bevorzugtesten 300 bis 2.500 Nukleotide und insbesondere 400 bis 2.000 Nukleotide.

In einer bevorzugten Ausführungsform umfasst die DNA-Ausgangssequenz innerhalb eines vorgegebenen Leserasters kein Stopp-Codon (UAA, UAG, UGA). Das Leseraster wird üblicherweise durch das Start-Codon einer Gen-Sequenz vorgegeben.

Bevorzugt codiert die DNA-Ausgangssequenz für ein Protein, besonders bevorzugt für ein Enzym. Vorzugsweise ist das Enzym ausgewählt aus der Gruppe bestehend aus 1. Oxidoreduktasen, 2. Transferasen, 3. Hydrolasen, 4. Lyasen, 5. Isomerasen und 6. Ligansen.

Bevorzugte Oxidoreduktasen sind ausgewählt aus der EC-Gruppe bestehend aus 1.1, 1.2, 1.3, 1.4, 1.5, 1.6, 1.7, 1.8, 1.9, 1.10, 1.11, 1.12, 1.13, 1.14, 1.15, 1.16, 1.17, 1.18, 1.19 und 1.97.

Bevorzugte Transferasen sind ausgewählt aus der EC-Gruppe bestehend aus 2.1, 2.2, 2.3, 2.4, 2.5, 2.6, 2.7, 2.8 und 2.9.

Bevorzugte Hydrolasen sind ausgewählt aus der EC-Gruppe bestehend aus 3.1, 3.2, 3.3, 3.4, 3.5, 3.6, 3.7, 3.8, 3.9, 3.10, 3.11 und 3.12.

Bevorzugte Lyasen sind ausgewählt aus der EC-Gruppe bestehend aus 4.1, 4.2, 4.3, 4.4, 4.5, 4.6 und 4.99.

Bevorzugte Isomerasen sind ausgewählt aus der EC-Gruppe bestehend aus 5.1, 5.2, 5.3, 5.4, 5.5 und 5.99.

Bevorzugte Ligasen sind ausgewählt aus der EC-Gruppe bestehend aus 6.1, 6.2, 6.3, 6.4 und 6.5.

Die von der International Union of Biochemistry and Molecular Biology (IUBMB) eingeführte EC-Nomenklatur ist dem Fachmann bekannt. Informationen hierzu finden sich auf Website des IUBMB.

In Schritt b) des erfindungsgemäßen Verfahrens wird die DNA-Ausgangssequenz in k Sequenzabschnitte unterteilt, wobei k mindestens 2 ist. Bevorzugt ist k $\geq 3$, bevorzugter k $\geq 4$, noch bevorzugter k $\geq 5$, am bevorzugtesten k $\geq 6$ und insbesondere k $\geq 8$.

Die Unterteilung erfolgt bevorzugt rein gedanklich, d.h. es findet bevorzugt keine Spaltung der DNA-Ausgangssequenz statt (z.B. mit Restriktionsendonukleasen). Bevorzugt werden die gedanklich unterteilten Sequenzabschnitte in Schritt c) durch PCR separat amplifiziert. Die Wahl geeigneter *Primer* ermöglicht die getrennte Amplifikation der einzelnen Sequenzabschnitte, wodurch letztlich einzelne Fragmente der DNA-Ausgangssequenz entstehen (welche mitunter Mutationen tragen).

Die Sequenzabschnitte werden so festgelegt, dass mindestens zwei Sequenzabschnitte jeweils mindestens eine Mutationsstelle umfassen. Die k Sequenzabschnitte können daher grundsätzlich in folgende Gruppen unterteilt werden:

- g Sequenzabschnitte $S_0$, welche keine Mutationsstelle umfassen; und
- h Sequenzabschnitte $S>_0$, welche mindestens eine Mutationsstelle umfassen.

Die Gruppe der h Sequenzabschnitte $S>_0$, welche mindestens eine Mutationsstelle umfassen, kann ihrerseits weiter unterteilt werden in

- i Sequenzabschnitte $S_1$, welche eine Mutationsstelle umfassen; und
- j Sequenzabschnitte $S>_1$, welche mehrere, bevorzugt 2 Mutationsstellen umfassen

Die Gesamtheit aller Sequenzabschnitte k setzt sich demnach wie folgt zusammen:

$$k = g + h = g + i + j.$$

Bevorzugt ist g > h, g = h oder g < h.

In einer bevorzugten Ausführungsform des erfindungsgemäßen Verfahrens ist g = 0 (dann gilt: k = h). In einer anderen bevorzugten Ausführungsform des erfindungsgemäßen Verfahrens ist $g \geq 1$, bevorzugter $g \geq 2$, noch bevorzugter $g \geq 3$, am bevorzugtesten $g \geq 4$ und insbesondere $g \geq 5$.

Bei dem erfindungsgemäßen Verfahren ist bevorzugt $h \geq 2$. Bevorzugt ist $h \geq 3$, bevorzugter $h \geq 4$, noch bevorzugter $h \geq 5$, am bevorzugtesten $h \geq 6$ und insbesondere $h \geq 7$.

Bevorzugt ist i > j, i = j oder i < j.

In einer bevorzugten Ausführungsform des erfindungsgemäßen Verfahrens ist i = 0 (dann gilt: h = j). In einer anderen bevorzugten Ausführungsform des erfindungsgemäßen Verfahrens ist $i \geq 1$, bevorzugter $i \geq 2$, noch bevorzugter $i \geq 3$, am bevorzugtesten $i \geq 4$ und insbesondere $i \geq 5$.

Bei dem erfindungsgemäßen Verfahren ist bevorzugt $j \geq 1$. Bevorzugt ist $j \geq 2$, bevorzugter $j \geq 3$, noch bevorzugter $j \geq 4$, am bevorzugtesten $j \geq 5$ und insbesondere $j \geq 6$.

Die Festlegung der relativen Lage der einzelnen Sequenzabschnitte auf der DNA-Ausgangs-sequenz kann grundsätzlich beliebig erfolgen. Da die Mutationen an den Mutationsstellen durch *Mismatch*-Positionen bei der Amplifikation durch PCR eingeführt werden, werden die Sequenzabschnitte bevorzugt so festgelegt, dass die Mutationsstellen dort lokalisiert sind, wo bei der PCR die Oligonukleotide *(Primer)* binden, also in der Nähe des 5'- bzw- 3'-Endes der jeweiligen Sequenzabschnitte.

Weitere Überlegungen können bei der Festlegung der relativen Lage der einzelnen Sequenzabschnitte eine Rolle spielen. Diese Überlegungen sind dem Fachmann geläufig und ergeben sich z.B. aus der Durchführung der PCR oder der späteren Weiterverarbeitung der Amplifikationsprodukte. So ist es bei der PCR zum Beispiel allgemein bevorzugt, wenn der GC-Gehalt innerhalb der Primersequenz 50% nicht übersteigt. Auch bestimmte Erkennungssequenzen von kommerziell erhältlichen Restriktionsendonukleasen können eine Rolle spielen, da mit diesen Enzymen z.B. anschließend *"sticky-ends"* erzeugt werden können, was für die spätere Ligation in Schritt d) von Vorteil sein kann. Grundsätzlich erlaubt das erfindungsgemäße Verfahren eine beliebige Aufteilung der DNA-Sequenz in Sequenzabschnitte. In der Regel bestimmt die Position der einzelnen Mutationsstellen die Unterteilung der DNA-Sequenz in Sequenzabschnitte.

Die Sequenzlänge der einzelnen Sequenzabschnitte $S_0$, $S_1$ bzw. $S>_1$ kann grundsätzlich frei gewählt werden.

Bevorzugt umfasst jeder Sequenzabschnitt mindestens 48 Nukleotide, bevorzugter mindestens 80 Nukleotide, noch bevorzugter mindestens 120 Nukleotide, am bevorzugtesten mindestens 150 Nukleotide und insbesondere mindestens 200 Nukleotide.

Bevorzugt umfasst jeder Sequenzabschnitt höchstens 10.000 Nukleotide, bevorzugter höchstens 8.000 Nukleotide, noch bevorzugter höchstens 7.000 Nukleotide, am bevorzugtesten höchstens 6.000 Nukleotide und insbesondere höchstens 5.000 Nukleotide.

Bei dem erfindungsgemäßen Verfahren fungieren die insgesamt wenigstens fünf verschiedenen Oligonukleotide üblicherweise als *Primer* bei der Amplifikation durch PCR.

Die Gesamtzahl der verschiedenen Oligonukleotide p beträgt bevorzugt $p \geq 10$, bevorzugter $p \geq 15$, noch bevorzugter $p \geq 20$, am bevorzugtesten $p \geq 30$ und insbesondere $p \geq 40$.

Die Oligonukleotide weisen jeweils bevorzugt eine Sequenzlänge von 15 bis 100 Nukleotiden auf, bevorzugter 20 bis 80, noch bevorzugter 25 bis 60, am bevorzugtesten 27 bis 50 und insbesondere 30 bis 45 auf.

Hinsichtlich einer ersten Eigenschaft können die Oligonukleotide grundsätzlich in folgende Gruppen unterteilt werden:

- Oligonukleotide, welche an das 5'-Ende eines Sequenzabschnitts auf der DNA-Ausgangssequenz binden, d.h. zum 5'-Ende eines Sequenzabschnitts auf der DNA-Ausgangssequenz (Mutterstrang) komplementär sind *(Match* oder *Mismatch)* (= *"sense-Primer" bzw. "5'-Primer");* und

- Oligonukleotide, welche an das 3'-Ende eines Sequenzabschnitts auf der DNA-Ausgangssequenz binden, d.h. zum 5'-Ende des komplementären Sequenzabschnitts auf der DNA-Ausgangssequenz (Tochterstrang) komplementär sind *(Match* oder *Mismatch)* (= "*antisense-Primer*" bzw. *"3'-Primer").*

Da für jeden zu amplifizierenden Sequenzabschnitt jeweils mindestens zwei Primer benötigt werden, nämlich jeweils ein *sense-Primer* und jeweils ein *antisense-Primer,* werden für die Amplifikation von insgesamt k Sequenzabschnitten auf der DNA-Ausgangssequenz üblicherweise mindestens k *sense-Primer* und k *antisense-Primer* benötigt, insgesamt also 2k verschiedene Oligonukleotide.

Da bei dem erfindungsgemäßen Verfahren an mindestens eine Mutationsstelle eines Sequenzabschnitts wenigstens zwei der Oligonukleotide anlagern können (vgl. Schritt c) (ii)), liegt bei diesen *sense-Primern* und/oder *antisense-Primern* eine Art Konkurrenzsituation vor. Neben den 2k verschiedenen Oligonukleotiden liegen daher zusätzlich noch insgesamt I verschiedene Oligonukleotide vor, welche zu mindestens einem der 2k Oligonukleotide (und ggf. auch untereinander) in Konkurrenz stehen, d.h. an dieselbe Bindungsstelle auf dem Sequenzabschnitt binden können.

Für die Gesamtzahl der verschiedenen Oligonukleotide p ergibt sich daher üblicherweise der folgende Zusammenhang: p = 2k + I.

Zum Zwecke der Beschreibung bedeutet in diesem Zusammenhang "Konkurrenz" nicht unbedingt, dass die einzelnen Oligonukleotide kompetitiv gleichzeitig nebeneinander vorliegen und um eine gemeinsame Bindungsstelle auf einem bestimmten Sequenzabschnitt konkurrieren müssen. So können die einzelnen Oligonukleotide auch einzeln in voneinander getrennten Gefäßen jeweils mit einem identischen Sequenzabschnitt in Kontakt gebracht werden und die so getrennt durch PCR erhaltenen Amplifikations-Produkte (Fragmente) später ggf. miteinander vermischt werden.

In einer bevorzugten Ausführungsform ist bei dem erfindungsgemäßen Verfahren die Anzahl der Oligonukleotide, die sich an einer Mutationsstelle anlagern können, identisch mit der Anzahl der Aminosäuren, die an der entsprechenden Position in der resultierenden Protein-Bibliothek erlaubt sind.

Bevorzugt werden bei dem erfindungsgemäßen Verfahren keine Gemische von Oligonukleotiden eingesetzt, die sich an die gleiche Bindungsstelle anlagern. Bevorzugt werden bei der Erzeugung der Sequenzabschnitts-Fragmente alle möglichen Kombinationen von sense- und antisense-Primern in separaten, unabhängigen PCR-Reaktionen eingesetzt.

In der einfachsten Ausführungsform des erfindungsgemäßen Verfahrens wird die DNA-Ausgangssequenz in zwei Sequenzabschnitte unterteilt (vgl. Schritt b)), so dass k = 2 ist. Für die Amplifikation dieser beiden Sequenzabschnitte werden 2k, also 4 verschiedene Oligonukleotide als *Primer* benötigt, von denen 2 Oligonukleotide als *sense-Primer* und die anderen 2 Oligonukleotide als *antisense-Primer* fungieren. In der einfachsten Ausführungsform des erfindungsgemäßen Verfahrens können sich an eine Mutationsstelle eines Sequenzabschnitts, der mindestens diese eine Mutationsstelle umfasst, 2 Oligonukleotide anlagern (Konkurrenzsituation), von denen das erste Oligonukleotid bereits unter den vorstehend erwähnten 2k Oligonukleotiden ist und das zweite Oligonukleotid mit I = 1 berücksichtigt wird. Für die Gesamtzahl der Oligonukleotide p ergibt sich somit p = 4+1 = 5.

In einer bevorzugten Ausführungsform des erfindungsgemäßen Verfahrens ist I ≥ 2, bevorzugter ≥ 5, ≥ 6 oder ≥ 7, noch bevorzugter ≥ 8, ≥ 9 oder ≥ 10, am bevorzugtesten ≥ 15, ≥ 20 oder ≥ 25, und insbesondere ≥ 30, ≥ 40 oder ≥ 50.

Hinsichtlich einer zweiten Eigenschaft können die Oligonukleotide grundsätzlich in folgende Gruppen unterteilt werden:

- Oligonukleotide, welche keine *Mismatch*-Positionen enthalten, d.h. bei der Amplifikation durch PCR keine Mutation erzeugen (= "*Match*-Oligonukleotide"); und

- Oligonukleotide, welche mindestens eine *Mismatch*-Position enthalten, d.h. bei der Amplifikation durch PCR mindestens eine Mutation erzeugen und demzufolge an eine Mutationsstelle anlagern können (= "*Mismatch*-Oligonukleotide").

Im Sinne der Beschreibung bedeutet bevorzugt "*Match*", dass das betreffende Nukleotid komplementär zur DNA-Ausgangssequenz ist, und *"Mismatch",* dass das betreffende Nukleotid nicht vollständig komplementär zur DNA-Ausgangssequenz ist.

In einer bevorzugten Ausführungsform des erfindungsgemäßen Verfahrens ist die Gesamtzahl der *Match*-Oligonukleotide größer als die Gesamtzahl der *Mismatch*-Oligonukleotide. In einer anderen bevorzugten Ausführungsform des erfindungsgemäßen Verfahrens ist die Gesamtzahl der *Match*-Oligonukleotide kleiner als die Gesamtzahl der *Mismatch*-Oligonukleotide.

Ein Fachmann erkennt, dass je nach Art der Sequenzabschnitte S für die Amplifikation durch PCR eine bestimmte Anzahl von *Match*- bzw. *Mismatch*-Oligonukleotiden erforderlich ist. Beispielsweise werden für die g Sequenzabschnitte $S_0$ der DNA-Ausgangssequenz, welche keine Mutationsstelle umfassen, bei der Amplifikation durch PCR 2g *Match*-Oligonukleotide benötigt. Analog werden für die i Sequenzabschnitte $S_1$, welche eine Mutationsstelle umfassen, (mindestens) i *Mismatch*-Oligonukleotide und i *Match*-Oligonukleotide benötigt.

Die *Mismatch*-Oligonukleotide können ihrerseits in folgende Gruppen unterteilt werden:

- *Mismatch*-Oligonukleotide, welche eine Mutation einführen (= *"single-Mismatch*-Oligonukleotide"); und
- *Mismatch*-Oligonukleotide, welche mehrere Mutationen einführen *("multiple-Mismatch*-Oligonukleotide").

In einer bevorzugten Ausführungsform des erfindungsgemäßen Verfahrens ist die Anzahl der *single-Mismatch*-Oligonukleotide größer als die Anzahl der *multiple-Mismatch-Oligonukleo*tide. In einer anderen bevorzugten Ausführungsform des erfindungsgemäßen Verfahrens ist die Anzahl der single-*Mismatch*-Oligonukleotide kleiner als die Anzahl der *multiple-Mismatch*-Oligonukleotide. In einer weiteren bevorzugten Ausführungsform werden auschließlich *single-Mismatch*-Oligonukleotide eingesetzt.

Die k Sequenzabschnitte enthalten die m Mutationsstellen. Bevorzugt sind die Mutationsstellen mehr oder weniger gleichmäßig über die Sequenzabschnitte verteilt. Bevorzugt enthält jeder Sequenzabschnitt unabhängig voneinander 0 bis 2 Mutationsstellen, bevorzugter 1 bis 2 Mutationsstellen, noch bevorzugter genau 2 Mutationsstellen.

In Schritt c) des erfindungsgemäßen Verfahrens werden die Sequenzabschnitte durch PCR amplifiziert. Die Amplifikation der unterschiedlichen mutierten Varianten der Sequenzabschnitte kann gemeinsam oder räumlich getrennt erfolgen. In einer bevorzugten Ausführungsform wird je Sequenzabschnitt mindestens eine eigene PCR durchgeführt, welche räumlich getrennt von der Amplifikation der übrigen Sequenzabschnitte erfolgen kann. Insofern ist es bedeutsam, wie viele Mutationsstellen auf einem Sequenzabschnitt vorliegen, da diese Mutationsstellen dann bei der PCR vorzugsweise auf dem gleichen Sequenzabschnitt vorliegen und von den übrigen Sequenzabschnitten und den darauf liegenden Mutationsstellen ggf. räumlich getrennt sind.

Für die PCR eignen sich grundsätzlich beliebige Polymerasen, z.B. die kommerziell erhältliche Taq-, die Vent- oder die Pfu-Polymerase.

In einer bevorzugten Ausführungsform werden bei der PCR Polymerasen eingesetzt, deren *proof-reading*-Funktion eingeschränkt oder vollständig unterdrückt ist ("fehlerhafte Polymerase "). Auf diese Weise werden infolge der PCR weitere Mutationen eingeführt, welche nicht zwingend an den Mutationsstellen vorliegen. Durch Verwendung solcher Polymerasen können Varianten erzeugt werden, deren Anzahl von Mutations-Positionen deutlich größer ist als die Anzahl der auf der DNA-Ausgangssequenz festgelegten Mutationsstellen.

In einer bevorzugten Ausführungsform erfolgt die Amplifikation der Sequenzabschnitte räumlich getrennt voneinander. Dazu werden vorzugsweise mehrere identische DNA-Ausgangssequenzen auf verschiedene Gefäße oder Kompartimente eines Gefäßes aufgeteilt und in jedem Gefäß / Kompartiment unter Einsatz geeigneter Oligonukleotide (mindestens ein *sense-Primer* und ein *antisense-Primer)* ein bestimmter Sequenzabschnitt amplifiziert. Die Abtrennung der so erhaltenen doppelsträngigen Amplifikations-Produkte (Fragmente), welche ggf. Mutationen tragen, von der DNA-Ausgangssequenz kann mit Hilfe üblicher Methoden erfolgen, beispielsweise durch Gelextraktion nach elektrophoretischer Trennung oder durch Gelfiltration oder Ionenaustausch-Chromatographie

In einer bevorzugten Ausführungsform erfolgt die Amplifikation der unterschiedlichen mutierten Varianten einzelner Sequenzabschnitte ebenfalls räumlich getrennt voneinander. Dazu werden die für einen bestimmten Sequenzabschnitt möglichen Oligonukleotid-Kombinationen einzeln für sich oder in zusammengefassten Gruppen in separaten Gefäßen / Kompartimenten für die PCR-Amplifikation eingesetzt.

Bevorzugt erfolgt die Amplifikation von mindestens 2 Varianten, bevorzugter mindestens 3 Varianten und insbesondere mindestens 4 Varianten mindestens eines Sequenzabschnitts, bevorzugt mindestens zweier Sequenzabschnitte und insbesondere mindestens dreier Sequenzabschnitte jeweils räumlich getrennt voneinander.

Bei dem erfindungsgemäßen Verfahren kann sich an jede Mutationsstelle mindestens eines der Oligonukleotiden anlagern. So wird gewährleistet, dass über Mismafch-Positionen Mutationen an den Mutationsstellen eingeführt werden können. Es ist auch möglich, dass mehrere Oligonukleotide an dieselbe Mutationsstelle anlagern können und in Konkurrenz zueinander stehen. Handelt es sich um *Mismatch*-Oligonukleotide, so fügen diese dann unterschiedliche Mutationen ein. Liegt ferner ein *Match*-Oligonukleotid im Konkurrenzverhältnis vor, so werden neben den Mutanten zusätzlich auch doppelsträngige Amplifikations-Produkte (Fragmente) erzeugt, welche der Ausgangssequenz entsprechen bzw. dazu vollständig komplementär sind. Bei dem erfindungsgemäßen Verfahren können sich an mindestens eine Mutationsstelle wenigstens zwei der Oligonukleotide anlagern.

Stehen mehrere Oligonukleotide im Hinblick auf eine Mutationsstelle in Konkurrenz zueinander, so können sie gemeinsam oder getrennt voneinander eingesetzt werden.

In einer bevorzugten Ausführungsform werden die in Konkurrenz stehenden Oligonukleotide auf verschiedene Gefäße aufgeteilt und in jedem Gefäß derselbe Sequenzabschnitt mit Hilfe des jeweiligen Oligonukleotids in Abwesenheit der anderen, in Konkurrenz stehenden Oligonukleotide amplifiziert.

Bei dieser Ausführungsform werden die für einen Sequenzabschnitt erzeugten doppelsträngigen Amplifikations-Produkte (Fragmente) vorzugsweise gemischt, ehe sie Schritt d) des erfindungsgemäßen Verfahrens zugeführt werden. Optional werden dabei die Amplifikationsprodukte vor dem Mischen quantifiziert.

Einzelne Schritte des erfindungsgemäßen Verfahrens können daher In verschiedener Hinsicht räumlich getrennt voneinander erfolgen. Einerseits können die einzelnen Sequenzabschnitte räumlich getrennt voneinander durch PCR amplifiziert werden, andererseits können aber ggf. zusätzlich auch diejenigen Sequenzabschnitte, welche Mutationsstellen enthalten, um die mehrere Oligonukleotide konkurrieren, mit jeweils einem einzelnen Oligonukleotid (oder auch nur einem Teil der insgesamt konkurrierenden Oligonukleotide) räumlich getrennt voneinander amplifiziert werden.

In einer bevorzugten Ausführungsform des erfindungsgemäßen Verfahrens werden die voneinander räumlich getrennt erzeugten DNA-Fragmente vor dem Mischen quantifiziert. Die Quantifizierung der DNA-Fragmente kann mit üblichen Methoden erfolgen. Zum Beispiel durch die densitometrlsche Auswertung von DNA-Agarose-Gelen, durch photometrische Bestimmung der Absorption der DNA-Lösung bei 260 nm oder durch Fluoreszenz-Methoden (zum Beispiel mit interkalierenden Fluoreszenzfarbstoffen wie Ethidiumbromid oder SYBR Green).

Bei dem erfindungsgemäßen Verfahren werden in den durch PCR erhaltenen doppelsträngigen Amplifikations-Produkten durch die Oligonukleotide an den Mutationsstellen über *Mismatch*-Positionen Mutationen eingeführt. Durch ein Oligonukleotid können auch an mehreren Positionen Mutationen eingeführt werden. In einer bevorzugten Ausführungsform werden durch mindestens ein, bevorzugt mindestens zwei, bevorzugter mindestens drei, noch bevorzugter mindestens vier, am bevorzugtesten mindestens fünf und insbesondere mindestens sechs Oligonukleotide jeweils mindestens zwei Mutationen eingeführt.

In einer bevorzugten Ausführungsform des erfindungsgemäßen Verfahrens werden Oligonukleotide, welche an derselben Mutationsstelle anlagern können, in einer PCR-Reaktion als Gemisch eingesetzt. In diesem Fall liegt eine unmittelbare Konkurrenzsituation vor. Da die Anzahl und Art der *Mismatch*-Positionen über die Affinität der Oligonukleotide an den Sequenzabschnitt entscheidet, können sich die in Konkurrenz stehenden Oligonukleotide in ihrer Affinität unterscheiden, was sich negativ auf die Gleichmäßigkeit der erzeugten doppelsträngigen Amplifikations-Produkte auswirken kann. Schlechter bindende Oligonukleotide dienen dann statistisch seltener als *Primer* und die entsprechenden Mutationen treten vergleichsweise seltener auf.

In einer anderen bevorzugten Ausführungsform des erfindungsgemäßen Verfahrens werden Oligonukleotide, welche an derselben Mutationsstelle anlagern können, In separaten PCR-Reaktionen eingesetzt und die erhaltenen PCR-Produkte werden im Anschluss gemischt. Besonders bevorzugt werden Oligonukleotide, welche sich an derselben Mutationsstelle anlagern können, in separaten PCR-Reaktionen eingesetzt, die erhaltenen PCR-Produkte werden im Anschluss in einem bestimmten Verhältnis gemischt und dadurch das Verhältnis der durch die Oligonukleotide eingeführten Mutationen in der Varianten-Bibliothek gesteuert.

In Schritt d) des erfindungsgemäßen Verfahrens werden die doppelsträngigen Amplifikationsprodukte (Fragmente) wieder in der richtigen Reihenfolge miteinander verknüpft. Dies kann auf unterschiedliche Weise geschehen.

In einer bevorzugten Ausführungsform erfolgt Schritt d) durch *Overlap Extension PCR.* Hierbei werden aufeinanderfolgende PCR-Fragmente so erzeugt, dass sie an ihren Enden identische Sequenzabschnitte aufweisen. Werden diese PCR-Fragmente in einer Folge-PCR gemeinsam eingesetzt, so können die komplementären Enden der Fragmente sich zusammenlagern und durch die eingesetzte DNA-Polymerese verlängert werden. Dadurch werden die PCR-Fragmente miteinander verknüpft. In einer solchen *Overlap Extension* PCR können mehrere PCR-Fragmente gleichzeitig miteinander verknüpft werden.

In einer anderen bevorzugten Ausführungsform werden mit Hilfe von Restriktionsendonukleasen an den Enden der zu verknüpfenden PCR-Fragmente *sticky ends* erzeugt, d.h. kurze Oligonukleotid-Überhänge, über welche die einzelnen Fragmente dann spezifisch mit Hilfe von DNR-Ligasen verknüpft werden können. Die Erkennungsstellen für die Restriktionsendonukleasen zur Erzeugung der *sticky ends* können über 5'-Überhänge der Primer In der PCR eingeführt werden. Werden Restriktionsendonukleasen des Typ II verwendet, welche die DNA innerhalb ihrer Erkennungssequenz schneiden, so solite beachtet werden, dass die nach Ligation entstehenden DNA-Sequenzen sich von der ursprünglichen Ausgangssequenz in der Regel unterscheiden werden. Entsprechende Translationen der DNA-Sequenzen in Proteine können zu Veränderungen in der Aminosäure-Sequenz führen. Besonders bevorzugt ist deswegen der Einsatz von Restriktionsenzymen des Typ IIS *(Outside Cutter).* Dies sind Restriktionsendonukleasen, die asymmetrische Erkennungssequenzen erkennen und außerhalb ihrer Erkennungssequenz schneiden. Mit Hilfe von *Outside Cuttern* können über 5'-Überhänge der Primer und anschließenden Restriktionsverdau zwei aufeinanderfolgende PCR-Fragmente einer DNA-Sequenz über *sticky ends* miteinander verknüpft werden, ohne dass die DNA-Ausgangssequenz an der Verbindungsstelle verändert wird. Es gibt auch *Outside-Cutter,* die *blunt ends* erzeugen. Verknüpft man zwei PCR-Fragmente über *blunt ends*, ist es möglich, zwei PCR-Fragmente so zu verbinden, dass oberhalb und unterhalb der Verbindungsstelle zwei Mutationen direkt nebeneinander liegen. So lassen sich beispielsweise in einer Protein-Sequenz zwei aufeinanderfolgende Aminosäure-Positionen mutieren, ohne das diese Teil einer gemeinsamen Mutationsstelle sein müssen. Werden die *blunt ends* von mindestens einem zu verknüpfenden Fragment in der ersten Ligationsreaktion nicht mit einem *Outside Cutter* eingeführt, so kann man durch eine Variation der Herangehensweise sogar 4 aufeinanderfolgende Mutationen an einer Verbindungsstelle erzeugen, ohne dass hierbei *Multiple-mismatch-Oligonukleotide* eingesetzt werden.

In einer bevorzugten Ausführungsform wird die Verknüpfung der Sequenzabschnitte stufenweise durchgeführt. Hierbei werden zunächst einzelne Sequenzabschnitte unabhängig voneinander verknüpft, so dass zunächst Zwischenprodukte erzeugt werden, die dann wiederum miteinander verknüpft werden. Wird eine DNA-Sequenz zum Beispiel in 8 Sequenzabschnitte aufgeteilt, können zunächst jeweils 2 Sequenzabschnitte miteinander verknüpft werden, so dass 4 Zwischenprodukte erhalten werden. Diese können dann wieder in zwei separaten Verknüpfungs-Reaktionen eingesetzt werden, so dass 2 wiederum verlängerte Zwischenprodukte entstehen, die dann zum Endprodukt verknüpft werden.

In einer bevorzugten Ausführungsform werden einzelne erzeugte Sequenzabschnitte, bzw. Sequenzabschnitts-Gemische oder bereits verknüpfte Sequenzabschnitte, bzw. Sequenzabschnitts-Gemische (Zwischenprodukte) durch eine zusätzliche PCR amplifiziert, um für die folgende Weiterbearbeitung die DNA-Menge zu erhöhen. Hierbei muss darauf geachtet werden, dass sich die hierfür verwendeten Primer auf der erneut zu amplifizierenden DNA-Sequenz außerhalb der gegebenenfalls vorhandenen Mutationsstellen anlagern.

Auf diese Weise kann gewährleistet werden, dass die Varianten der Varianten-Bibliothek (verknüpfte DNA-Sequenzen) im Wesentlichen die gleiche Sequenzlänge wie die DNA-Ausgangssequenz aufweisen. In einer bevorzugten Ausführungsform weicht die Sequenzlänge der erhaltenen DNA-Sequenzen (Varianten) um höchstens 10% von der Sequenzlänge der DNA-Ausgangssequenz ab, bevorzugter um höchstens 7,5%, noch bevorzugter um höchstens 5%, am bevorzugtesten um höchstens 2,5% und insbesondere um höchstens 1%. In einer besonders bevorzugten Ausführungsform ist die Sequenzlänge der erhaltenen DNA-Sequenzen (Varianten) im Wesentlichen identisch mit der Sequenzlänge der DNA-Ausgangssequenz.

Bevorzugt kodieren die DNA-Sequenzen der Varianten-Bibliothek für Proteine oder für Teile von Proteinen.

Zur Vereinzelung von Varianten der Bibliothek, zur Expression der Bibliothek und /oder zur Selektion oder zum Screening der Varianten-Bibliothek kann es notwendig sein, die Varianten-Bibliothek in einem Organismus zu überführen. In einer bevorzugten Ausführungsform umfasst das erfindungsgemäße Verfahren daher den Schritt:

e) Klonieren der Varianten in einen geeigneten Vektor.

Vektoren sind Transportmittel zur Übertragung von Fremd-Nukleinsäuren in eine Empfängerzelle. Vektoren können zum Beispiel Plasmide, Viren oder Cosmide sein. Entsprechende Klonierungsverfahren zum Einbringen der Varianten-Bibliothek in einen Vektor sind dem Fachmann hinlänglich bekannt.

Ein Empfänger-Organismus kann z. B. mit dem Vektor transformiert werden, um im Anschluss die korrespondierenden Proteine zu exprimieren. Das erfindungsgemäße Verfahren umfasst daher bevorzugt die Schritte:

f) Transfizieren des Vektors in einen geeigneten Organismus; und

g) Exprimieren der durch die jeweiligen Varianten kodierten Proteine.

Entsprechende Verfahren zur rekombinanten Expression von Proteinen mit Hilfe von Wirtszellen sind dem Fachmann hinlänglich bekannt.

Die Expression der Proteine kann jedoch alternativ auch zellfrei erfolgen.

Entsprechende Verfahren zur rekombinanten Expression von Proteinen durch zellfreie *invitro* Translations-Extrakte sind dem Fachmann hinlänglich bekannt.

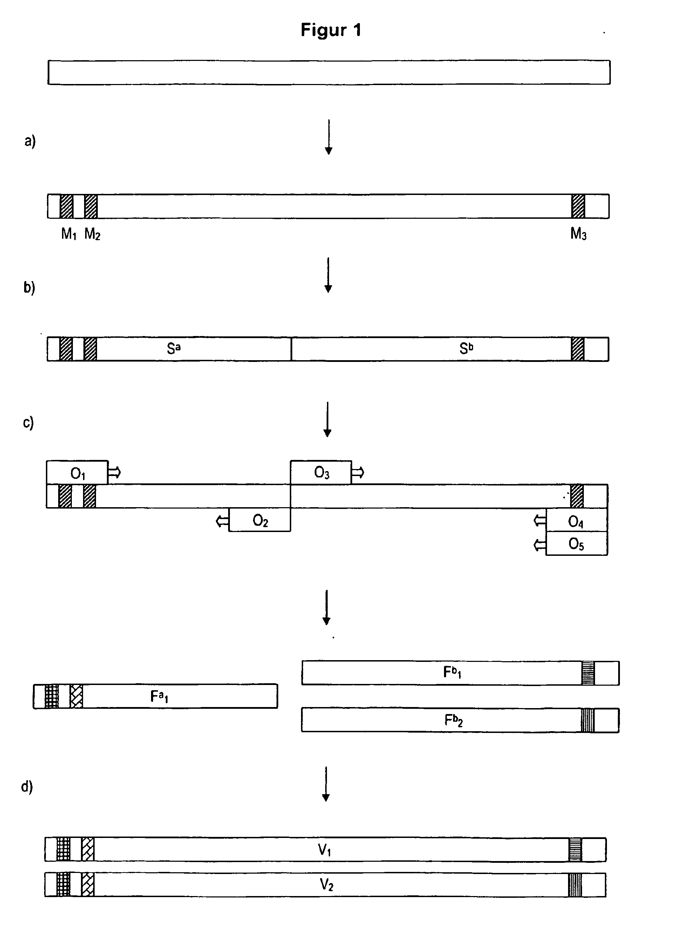

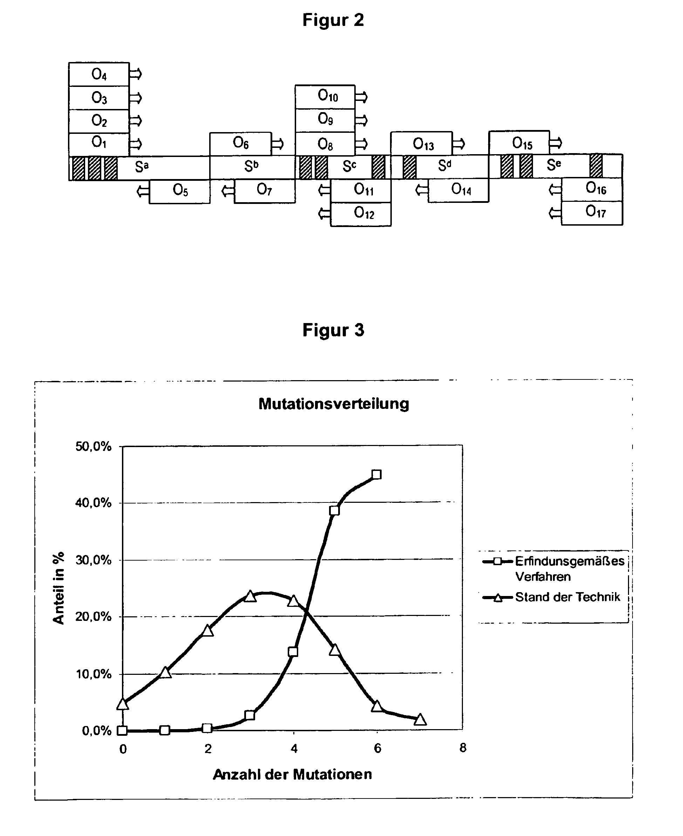

Das erfindungsgemäße Verfahren wird nachfolgend anhand von Figuren 1 und 2 näher erläutert.

Figur 1 zeigt schematisch eine besonders einfache Ausführungsform des erfindungsgemäßen Verfahrens. In Schritt a) werden auf der DNA-Ausgangssequenz drei Mutationsstellen $M_1$, $M_2$ und $M_3$ ausgewählt. In Schritt b) wird die DNA-Ausgangssequenz in zwei Sequenzabschnitte $S^a$ und $S^b$ unterteilt, wobei der Sequenzabschnitt $S^a$ die Mutationsstellen $M_1$ und $M_2$ und der Sequenzabschnitt $S^b$ die Mutationsstelle $M_3$ enthält. Bei dieser beispielhaft erläuterten Variante des Verfahrens tritt kein Sequenzabschnitt auf, welcher keine einzige Mutation enthält, was jedoch erfindungsgemäß möglich ist. In Schritt c) werden die beiden Sequenzabschnitte $S^a$ und $S^b$ mit Hilfe von den fünf Oligonukleotiden $O_1$, $O_2$. $O_3$, $O_4$ und $O_5$ amplifiziert. Dabei handelt es sich bei den Oligonukleotiden $O_1$ und $O_3$ um *sense-Primer* und bei den Oligonukleotiden $O_2$, $O_4$ und $O_5$ um *antisense-Primer,* was durch die Pfeile und die Anordnung ober- und unterhalb der Ausgangssequenz angedeutet ist. Ein Fachmann erkennt, dass die antisense-Primer an den zur DNA-Ausgangssequenz komplementären DNA-Strang binden. $O_1$, $O_4$ und $O_5$ sind Mismatch-Oligonukleotide und binden jeweils an Mutationsstellen, $O_2$ und $O_3$ sind Match-Oligonukleotide. $O_1$ führt an den Mutationsstellen $M_1$ und $M_2$ jeweils eine Mutation ein, $O_4$ und $O_5$ stehen in Konkurrenz zueinander und führen an der Mutationsstelle $M_3$ jeweils eine unterschiedliche Mutation ein. So entsteht durch Ampflifizieren von Sequenzabschnitt $S^a$ ein einzelnes Fragment $F^a_1$, welches zwei Mutationen aufweist, und durch Amplifizieren von Sequenzabschnitt $S^b$ entstehen zwei Fragmente $F^b$ und $F^b_2$, welche jeweils eine Mutation aufweisen, jedoch voneinander verschieden sind. Die Verknüpfung des Fragments $F^a$, mit dem Fragment $F^b$, liefert Variante V1 und die Verknüpfung des Fragments $F^a_1$ mit dem Fragment $F^b_2$ liefert Variante $V_2$ der Varianten-Bibliothek. Auf diese Weise wird durch das erfindungsgemäße Verfahren eine Varianten-Bibliothek von zwei Varianten erzeugt, welche sich beide von der DNA-Ausgangssequenz (Wildtyp-Sequenz) durch je genau drei Mutationen unterscheiden.

Figur 2 illustriert schematisch eine komplexere Situation, wie sie beim erfindungsgemäßen Verfahren auftreten kann. So ist die DNA-Ausgangssequenz hier in fünf Sequenzabschnitte $S^a$, $S^b$, $S^c$, $S^d$ und $S^e$ unterteilt. Sequenzabschnitte $S^a$, $S^c$ und $S^e$ umfassen jeweils drei Mutationsstellen, Sequenzabschnitt $S^b$ umfasst keine Mutationsstelle und Sequenzabschnitt $S^d$ umfasst eine Mutationsstelle. Oligonukleotide $O_1$ bis $O_4$, $O_6$, $O_8$ bis $O_{10}$, $O_{13}$ und $O_{15}$ sind *sense-Primer* und Oligonukleotide $O_5$, $O_7$, $O_{11}$, $O_{12}$, $O_{14}$, $O_{16}$ und $O_{17}$ sind *antisense-Primer.* Oligonukleotide $O_1$ bis $O_4$, $O_8$ bis $O_9$, $O_{11}$ und $O_{12}$, und $O_{16}$ und $O_{17}$ konkurrieren jeweils miteinander um Mutationsstellen. Zusammen mit $O_{13}$ handelt es sich um *Mismatch*-Oligonukleotide. $O_5$ bis $O_7$ und $O_{14}$ sind *Match*-Oligonukleotide.

Eine besondere Eigenschaft des erfindungsgemäßen Verfahrens kann im Zusammenhang mit den vier Oligonukleotiden $O_1$, $O_2$, $O_3$ und $O_4$ in Figur 2 erläutert werden. Es ist möglich, dass alle vier Oligonukleotide verschiedene *Mismatch*-Positionen für alle drei Mutationsstellen auf Sequenzabschnitt $S^a$ enthalten. Es ist aber auch möglich, dass die Anzahl der Mismatch-Positionen variiert, dass z.B. $O_1$ an allen drei Mutationsstellen Mutationen einführt, $O_2$ lediglich an zwei Mutationsstellen, $O_3$ an nur einer Mutationsstelle und $O_4$ an einer anderen oder aber an keiner Mutationsstelle ($O_4$ ist in letzterem Fall kein *Mismatch*-Oligonukleotid, sondern ein *Match*-Oligonukleotid). Durch geeignete Wahl der Oligonukleotide kann so die Mutantenverteilung von Anfang an sehr genau festgelegt werden, was einen besonderen Vorteil des erfindungsgemäßen Verfahrens ausmacht.

Bei dem erfindungsgemäßen Verfahren liegt wenigstens eine Mutationsstelle vor, um welche wenigstens 2 Oligonukleotide miteinander konkurrieren, von denen wenigstens 1 Oligonukleotid, vorzugsweise beide Oligonukleotide *Mismatch*-Oligonukleotide sind.

In einer bevorzugten Ausführungsform liegt bei dem erfindungsgemäßen Verfahren wenigstens eine Mutationsstelle vor, um welche wenigstens 3 Oligonukleotide miteinander konkurrieren, von denen wenigstens 2 Oligonukleotide, vorzugsweise alle Oligonukleotide, *Mismatch*-Oligonukleotide sind.

In einer anderen bevorzugten Ausführungsform liegt bei dem erfindungsgemäßen Verfahren wenigstens eine Mutationsstelle vor, um welche wenigstens 4 Oligonukleotide miteinander konkurrieren, von denen wenigstens 3 Oligonukleotide, vorzugsweise alle Oligonukleotide, *Mismatch*-Oligonukleotide sind.

In einer weiteren bevorzugten Ausführungsform liegt bei dem erfindungsgemäßen Verfahren wenigstens eine Mutationsstelle vor, um welche wenigstens 5 Oligonukleotide miteinander konkurrieren, von denen wenigstens 4 Oligonukleotide, vorzugsweise alle Oligonukleotide, *Mismatch*-Oligonukleotide sind.

Bevorzugt liegen bei dem erfindungsgemäßen Verfahren wenigstens zwei, bevorzugter wenigstens drei, noch bevorzugter wenigstens vier, am bevorzugtesten wenigstens fünf und insbesondere wenigstens sechs Mutationsstellen vor, um die jeweils mehrere Oligonukleotide miteinander konkurrieren, von denen bevorzugt jeweils wenigstens ein Oligonukleotid, bevorzugter alle Oligonukleotide, *Mismatch*-Oligonukleotide sind.

Weitere bevorzugte Ausführungsformen $A_1$ bis $A_{30}$ des erfindungsgemäßen Verfahrens sind in nachfolgender Tabelle zusammengefasst:

|  | $A_1$ | $A_2$ | $A_3$ | $A_4$ | $A_5$ | $A_6$ | $A_7$ | $A_8$ | $A_9$ | $A_{10}$ |
|---|---|---|---|---|---|---|---|---|---|---|
| $M^{\geq 2}$ | ≥ 2 | ≥ 2 | ≥ 2 | ≥ 2 | ≥ 2 | ≥ 3 | ≥ 3 | ≥ 3 | ≥ 3 | ≥ 3 |
| $M^{\geq 3}$ | ≥ 1 | ≥ 1 | ≥ 1 | ≥ 1 | ≥ 1 | ≥ 1 | ≥ 1 | ≥ 1 | ≥ 1 | ≥ 1 |
| $M^{\geq 24}$ | ≥ 0 | ≥ 1 | ≥ 1 | ≥ 1 | ≥ 1 | ≥ 0 | ≥ 1 | ≥ 1 | ≥ 1 | ≥ 1 |
| $M^{\geq 5}$ | ≥ 0 | ≥ 0 | ≥ 1 | ≥ 1 | ≥ 1 | ≥ 0 | ≥ 0 | ≥ 1 | ≥ 1 | ≥ 1 |
| $M^{\geq 6}$ | ≥ 0 | ≥ 0 | ≥ 0 | ≥ 1 | ≥ 1 | ≥ 0 | ≥ 0 | ≥ 0 | ≥ 1 | ≥ 1 |
| $M^{\geq 7}$ | ≥ 0 | ≥ 0 | ≥ 0 | ≥ 0 | ≥ 1 | ≥ 0 | ≥ 0 | ≥ 0 | ≥ 0 | ≥ 1 |

|  | $A_{11}$ | $A_{12}$ | $A_{13}$ | $A_{14}$ | $A_{15}$ | $A_{16}$ | $A_{17}$ | $A_{18}$ | $A_{19}$ | $A_{20}$ |
|---|---|---|---|---|---|---|---|---|---|---|
| $M^{\geq 2}$ | ≥ 4 | ≥ 4 | ≥ 4 | ≥ 4 | ≥ 4 | ≥ 2 | ≥ 2 | ≥ 2 | ≥ 2 | ≥ 2 |
| $M^{\geq 23}$ | ≥ 1 | ≥ 1 | ≥ 1 | ≥ 1 | ≥ 1 | ≥ 2 | ≥ 2 | ≥ 2 | ≥ 2 | ≥ 2 |
| $M^{\geq 4}$ | ≥ 0 | ≥ 1 | ≥ 1 | ≥ 1 | ≥ 1 | ≥ 0 | ≥ 1 | ≥1 | ≥ 1 | ≥ 1 |
| $M^{\geq 5}$ | ≥ 0 | ≥ 0 | ≥ 1 | ≥ 1 | ≥ 1 | ≥ 0 | ≥ 0 | ≥ 1 | ≥ 1 | ≥ 1 |
| $M^{\geq 6}$ | ≥ 0 | ≥ 0 | ≥ 0 | ≥ 1 | ≥ 1 | ≥ 0 | ≥ 0 | ≥ 0 | ≥ 1 | ≥ 1 |
| $M^{\geq 7}$ | ≥ 0 | ≥ 0 | ≥ 0 | ≥ 0 | ≥ 1 | ≥ 0 | ≥ 0 | ≥ 0 | ≥ 0 | ≥ 1 |

|  | $A_{21}$ | $A_{22}$ | $A_{23}$ | $A_{24}$ | $A_{25}$ | $A_{26}$ | $A_{27}$ | $A_{28}$ | $A_{29}$ | $A_{30}$ |
|---|---|---|---|---|---|---|---|---|---|---|
| $M^{\geq 2}$ | ≥ 3 | ≥ 3 | ≥ 3 | ≥ 3 | ≥ 3 | ≥ 3 | ≥ 3 | ≥ 3 | ≥ 3 | ≥ 3 |
| $M^{\geq 3}$ | ≥ 2 | ≥ 2 | ≥ 2 | ≥ 2 | ≥ 2 | ≥ 3 | ≥ 3 | ≥ 3 | ≥ 3 | ≥ 3 |

(fortgesetzt)

| | $A_{21}$ | $A_{22}$ | $A_{23}$ | $A_{24}$ | $A_{25}$ | $A_{26}$ | $A_{27}$ | $A_{28}$ | $A_{29}$ | $A_{30}$ |
|---|---|---|---|---|---|---|---|---|---|---|
| $M^{\geq 4}$ | $\geq 0$ | $\geq 1$ | $\geq 1$ | $\geq 1$ | $\geq 1$ | $\geq 0$ | $\geq 1$ | $\geq 1$ | $\geq 1$ | $\geq 1$ |
| $M^{\geq 5}$ | $\geq 0$ | $\geq 0$ | $\geq 1$ | $\geq 1$ | $\geq 1$ | $\geq 0$ | $\geq 0$ | $\geq 1$ | $\geq 1$ | $\geq 1$ |
| $M^{\geq 6}$ | $\geq 0$ | $\geq 0$ | $\geq 0$ | $\geq 1$ | $\geq 1$ | $\geq 0$ | $\geq 0$ | $\geq 0$ | $\geq 1$ | $\geq 1$ |
| $M^{\geq 7}$ | $\geq 0$ | $\geq 0$ | $\geq 0$ | $\geq 0$ | $\geq 1$ | $\geq 0$ | $\geq 0$ | $\geq 0$ | $\geq 0$ | $\geq 1$ |

Dabei bedeutet "$M^{\geq 2}$" eine Mutationsstelle, um welche mindestens zwei Oligonukleotide miteinander konkurrieren, "$M^{\geq 3}$" eine Mutationsstelle, um welche mindestens drei Oligonukleotide miteinander konkurrieren, usw. Diese Definition ist kumulativ, da eine Mutationsstelle, um die z.B. vier Oligonukleotide miteinander konkurrieren, gleichzeitig eine Mutationsstelle ist, um die "mindestens 2", "mindestens 3" und "mindestens 4" Oligonukleotide miteinander konkurrieren.

Demnach liegt z.B. gemäß Ausführungsform $A_4$ eine Mutationsstelle vor, um welche wenigstens 6 Oligonukleotide miteinander konkurrieren und eine Mutationsstelle, um welche wenigstens 2 Oligonukleotide miteinander konkurrieren. Analog liegt gemäß Ausführungsform $A_{28}$ eine Mutationsstelle vor, um welche wenigstens fünf Oligonukleotide miteinander konkurrieren, und zwei Mutationsstellen, um die jeweils mindestens drei Oligonukleotide miteinander konkurrieren.

Bevorzugt beträgt die Anzahl der durch das erfindungsgemäße Verfahren erzeugten unterschiedlichen Varianten in der Varianten-Bibliothek mindestens $10^1$, bevorzugter mindestens $10^2$, noch bevorzugter mindestens $10^3$, am bevorzugtesten mindestens $10^4$ und insbesondere mindestens $10^5$. Jede einzelne Variante ist dabei mindestens einmal vertreten, kann jedoch auch mehrfach vertreten sein.

Die Anzahl der möglichen Varianten lässt sich durch einfache Überlegungen abschätzen. Im Zusammenhang mit Figur 2 sind ergeben sich für die drei Mutationsstellen auf Sequenzabschnitt $S^a$ durch die vier Oligonukleotide $O_1$, $O_2$, $O_3$ und $O_4$ vier Möglichkeiten; für die beiden links dargestellten Mutationsstellen auf Sequenzabschnitt $S^c$ durch die beiden Oligonukleotide $O_8$, $O_9$ und $O_{10}$ drei Möglichkeiten, für die links dargestellte Mutationsstelle auf Sequenzabschnitt $S^c$ wegen der Oligonukleotide $O_{11}$ und $O_{12}$ zwei Möglichkeiten und für die rechts dargestellte Mutationsstelle auf Sequenzabschnitt $S^e$ wegen der Oligonukleotide $O_{16}$ und $O_{17}$ ebenfalls 2 Möglichkeiten. Für die übrigen Mutationsstellen ergibt sich in Ermangelung einer Konkurrenzsituation mehrerer Oligonukleotide lediglich eine einzige Möglichkeit. Die Gesamtzahl der möglichen Varianten ergibt somit zu 4 x 3 x 2 x 2 = 48.

Bevorzugt kann die Anzahl K der insgesamt in der Varianten-Bibliothek vorhandenen Varianten nach folgender Formel exakt berechnet werden: $K = (N_1 \times N_2 \times ... \times N_m) - W$, wobei m die Anzahl der Mutationsstellen ist, N die Anzahl der zugelassenen Varianten inklusive der gegebenenfalls zugelassenen Wildtyp-Sequenz für eine bestimmte Mutationsstelle ist und W der Anteil der unmutierten Sequenzen (Wildtyp-Sequenzen) der Varianten-Bibliothek ist.

In einer bevorzugten Ausführungsform wird das erfindungsgemäße Verfahren in einer Weise durchgeführt, dass in der Varianten-Bibliothek weniger als 1,0%, bevorzugter weniger als 0,5%, noch bevorzugter weniger als 0,1%, am bevorzugtesten weniger als 0,05% und insbesondere weniger als 0,01% DNA-Ausgangssequenzen (Wildtyp-Sequenzen) enthalten sind. In einer besonders bevorzugten Ausführungsform wird das erfindungsgemäße Verfahren in einer Weise durchgeführt, dass in der Varianten-Bibliothek überhaupt keine (DNA-Ausgangssequenzen) Wildtyp-Sequenzen enthalten sind.

Bevorzugt kann der Anteil der unmutierten Sequenzen W (Wildtyp-Sequenzen) der Varianten-Bibliothek über folgende Formel berechnet werden: $W = A_1 \times A_2 \times ... \times A_m$, wobei m die Anzahl der Mutationsstellen und A der für eine bestimmte Mutationsstelle eingesetzte prozentuale Anteil der nicht mutierenden Oligonukleotide (*Match*-Oligonukleotide) ist. Umfasst die DNA-Ausgangssequenz beispielsweise 3 Mutationsstellen, wobei um die ersten beiden Mutationsstellen 7 Oligonukleotide konkurrieren, von denen 6 Oligonukleotide *Mismatch*-Oligonukleotide sind und ein Oligonukleotid ein *Match*-Oligonukleotid ist, und wobei um die dritte Mutationsstelle fünf Oligonukleotide konkurrieren, von denen 4 Oligonukleotide *Mismatch*-Oligonukleotide sind und ein Oligonukleotid ein *Match*-Oligonukleotid ist, so ergibt sich: W = 1/7 x 1/7 x 1/5 = 1/245 = 0.41%.

In einer bevorzugten Ausführungsform enthält die Varianten-Bibliothek Varianten, welche genauso viele Mutationen wie Mutationsstellen aufweisen. Bevorzugter ist in der durch das erfindungsgemäße Verfahren erzeugten Varianten-Bibliothek die Anzahl der Varianten, die genauso viele Mutationen wie Mutationsstellen aufweisen, größer als die Anzahl der Varianten, deren Anzahl von Mutationen um 1 kleiner ist als die Anzahl der Mutationsstellen. Noch bevorzugter ist die Anzahl der Varianten, deren Anzahl von Mutationen um 1 kleiner ist als die Anzahl der Mutationsstellen, größer als die Anzahl der Varianten, deren Anzahl von Mutationen um 2 kleiner ist als die Anzahl der Mutationsstellen. Besonders bevorzugt gilt bei insgesamt m Mutationsstellen in der DNA-Ausgangssequenz, dass die Anzahl der Varianten, deren Anzahl von Mutationen q beträgt, genauso groß oder größer ist als die Anzahl der Varianten, deren Anzahl von Mutationen q-1 ist mit q = 1 bis m.

In dieser Hinsicht kann die Mutantenverteilung durch das Verhältnis von *Mismatch*- und *Match*-Oligonukleotiden eingestellt werden. Konkurrieren beispielsweise zwei *Mismatch*-Oligonukleotide und ein *Match*-Oligonuklevtid um dieselbe Mutationsstelle, so erzeugen die *Mismatch*-Oligonukleotide bei der PCR Mutationen, das *Match*-Oligonukleotid hingegen nicht.

Bevorzugt beträgt das relative Verhältnis der Varianten, die genauso viele Mutationen wie Mutationsstellen aufweisen zu den Varianten, deren Anzahl von Mutationen um 1 kleiner ist als die Anzahl der Mutationsstellen, wenigstens 55:45, bevorzugter wenigstens 60:40, noch bevorzugter wenigstens 65:35, am bevorzugtesten wenigstens 70:30 und insbesondere wenigstens 75:25.

Bevorzugt wird der Anteil der Varianten-Fraktion in der Varianten-Bibliothek mit der Anzahl der Mutationen der Varianten der Fraktion größer.

Bevorzugt ist die größte Varianten-Fraktion die Fraktion mit der größten Anzahl von Mutationen, so dass die Bibliothek keine Varianten-Fraktion enthält, die mehr Mutationen aufweist als die größte Varianten-Fraktion.

Das erfindungsgemäße Verfahren wird so durchgeführt, dass für mindestens 75%, bevorzugt mindestens 80%, bevorzugter mindestens 85%, noch bevorzugter mindestens 90% und am bevorzugtesten mindestens 95% der erzeugten

Varianten in der Varianten-Bibliothek die Anzahl der Mutationen pro Variante in einem Bereich N liegt, wobei $X \geq N \geq (X-3)$, wobei $X \in N$ und $25 > X > 3$. Bevorzugt liegt für mindestens 75%, bevorzugter mindestens 80%, noch bevorzugter mindestens 85%, am bevorzugtesten mindestens 90% und insbesondere mindestens 95% der erzeugten Varianten in der Varianten-Bibliothek die Anzahl der Mutationen pro Variante in einem Bereich N, wobei $X \geq N \geq (X-2)$, wobei $X \in N$ und $20 > X > 2$. Bevorzugter liegt für mindestens 75%, bevorzugter mindestens 80%, noch bevorzugter mindestens 85%, am bevorzugtesten mindestens 90% und insbesondere mindestens 95% der erzeugten Varianten in der Varianten-Bibliothek die Anzahl der Mutationen pro Variante in einem Bereich N, wobei $X \geq N \geq (X-1)$, wobei $X \in N$ und $20 > X > 1$.

In einer bevorzugten Ausführungsform wird das erfindungsgemäße Verfahren so durchgeführt, dass die Anzahl der Mutationen pro Variante in allen Varianten der Varianten-Bibliothek gleich ist.

Figur 3 zeigt die Mutationsverteilungen von zwei Varianten-Bibliotheken. Die eine Mutations-verteilung entspricht einer typischen Mutationsverteilung, wie sie bei der Durchführung des erfindungsgemäßen Verfahrens erzeugt werden kann. Die zweite Mutationsverteilung entspricht einer Mutationsverteilung wie sie typischerweise durch herkömmliche Verfahren des Stand der Technik wie zum Beispiel *Error-Prone* PCR oder *Massive Mutagenesis* erzeugt werden kann.

Ein weiterer Gegenstand der Erfindung betrifft eine Varianten-Bibliothek einer DNA-Sequenz, welche nach dem erfindungsgemäßen Verfahren erhältlich ist.

Ein weiterer Gegenstand der Erfindung betrifft eine Varianten-Bibliothek einer DNA-Sequenz, welche mindestens 3 Mutationsstellen aufweist, wobei

- in der Varianten-Bibliothek in Bezug auf jede Mutationsstelle jeweils mindestens eine Variante enthalten ist, welche an mindestens dieser Mutationsstelle mindestens eine Mutation aufweist,
- in der Varianten-Bibliothek keine Variante enthalten ist, die mehr als 2 Mutationen, bevorzugt mehr als 1 Mutation mehr aufweist als die Anzahl der Mutationen der größten Fraktion der Varianten, und
- die Anzahl der Mutationen der größten Fraktion der Varianten $\geq 3$ ist.

Die erfindungsgemäße Varianten-Bibliothek ist durch eine besondere Verteilung der Mutationen gekennzeichnet, wobei alle Varianten mit einer bestimmten Anzahl von Mutationen in einer Fraktion zusammengefasst werden. Die größte Fraktion der Varianten weist eine Anzahl d von Mutationen auf. Rein theoretisch gibt es dann kleinere Fraktionen der Varianten, die d-1, d-2, d-3, ... Mutationen aufweisen, und kleinere Fraktionen der Varianten, die d+1, d+2, d+3, ... Mutationen aufweisen. Die erfindungsgemäße Varianten-Bibliothek ist dadurch gekennzeichnet, dass sie keine Varianten enthält, welche d+3 oder mehr Mutationen aufweisen. Bevorzugt ist die erfindungsgemäße Varianten-Bibliothek dadurch gekennzeichnet, dass sie keine Varianten enthält, welche d+2 oder mehr Mutationen aufweisen. Besonders bevorzugt ist die erfindungsgemäße Varianten-Bibliothek dadurch gekennzeichnet, dass sie keine Varianten enthält, welche d+1 oder mehr Mutationen aufweisen.

In der erfindungsgemäßen Varianten-Bibliothek enthält die Fraktion der Varianten, welche d+2 Mutationen aufweist, höchstens 10% und bevorzugt höchstens 5%.

Besonders bevorzugte Ausführungsformen $B_1$ bis $B_{20}$ der erfindungsgemäßen Varianten-Bibliothek sind in nachfolgender Tabelle zusammengefasst, wobei die größte Fraktion der Varianten eine Anzahl m von Mutationen aufweist und die Prozentangaben jeweils den prozentualen Gehalt der unterschiedlichen Varianten der Fraktion in Bezug auf die Gesamtzahl aller unterschiedlichen Varianten in der Bibliothek angeben:

| | d | d+1 | d+2 | | | d | d+1 | d+2 |
|---|---|---|---|---|---|---|---|---|
| $B_1$ | ≥ 25% | ≤ 20% | ≤ 10% | | $B_{11}$ | ≥ 35% | ≤ 20% | ≤ 10% |
| $B_2$ | ≥ 25% | ≤ 15% | ≤ 10% | | $B_{12}$ | ≥ 35% | ≤ 15% | ≤ 10% |
| $B_3$ | ≥ 25% | ≤10% | ≤ 5% | | $B_{13}$ | ≥ 35% | ≤ 10% | ≤ 5% |
| $B_4$ | ≥ 25% | ≤ 5% | 1 % | | $B_{14}$ | ≥ 35% | ≤ 5% | ≤ 1 % |
| $B_5$ | ≥ 25% | ≤ 1% | ≤ 0,5% | | $B_{15}$ | ≥ 35% | ≤ 1% | ≤ 0,5% |
| $B_6$ | ≥ 30% | ≤ 20% | ≤ 10% | | $B_{16}$ | ≥ 40% | ≤ 20% | ≤ 10% |
| $B_7$ | ≥ 30% | ≤ 15% | ≤ 10% | | $B_{17}$ | ≥ 40% | ≤ 15% | ≤ 10% |
| $B_8$ | ≥ 30% | ≤ 10% | ≤ 5% | | $B_{18}$ | ≥ 40% | ≤ 10% | ≤ 5% |
| $B_9$ | ≥ 30% | ≤ 5% | ≤ 1% | | $B_{19}$ | ≥ 40% | ≤ 5% | ≤ 1% |
| $B_{10}$ | ≥ 30% | ≤ 1% | ≤ 0,5% | | $B_{20}$ | ≥ 40% | ≤ 1% | ≤ 0,5% |

Die Anzahl der Mutationen d der größten Fraktion beträgt dabei bevorzugt mindestens 3, mindestens 4, mindestens 5 oder mindestens 6, bevorzugter mindestens 7, mindestens 8, mindestens 9 oder mindestens 10, noch bevorzugter mindestens 11, mindestens 12, mindestens 13 oder mindestens 14, am bevorzugtesten mindestens 15, mindestens 16, mindestens 17 oder mindestens 18 und insbesondere mindestens 19, mindestens 20, mindestens 21 oder mindestens 22.

Weitere bevorzugte Ausführungsformen der erfindungsgemäßen Varianten-Bibliothek ergeben sich aus den vorstehend beschriebenen bevorzugten Ausführungsformen des erfindungsgemäßen Verfahrens. Diese bevorzugten Ausführungsformen gelten entsprechend und werden daher an dieser Stelle nicht wiederholt.

Die nachfolgenden Beispiele dienen zur näheren Erläuterung der Erfindung, sind jedoch nicht einschränkend auszulegen:

Beispiel 1:

Im Folgenden ist die Erstellung einer Varianten-Bibliothek mit Hilfe des erfindungsgemäßen Verfahrens beschrieben. Die Ausgangssequenz ist ein Enzym-Gen. Innerhalb der Gen-Sequenz wurden 6 Mutationsstellen (1-6) ausgewählt, die auf insgesamt 3 Sequenzabschnitten liegen. Ein vierter Sequenzabschnitt enthält keine Mutationsstelle.

| Sequenzabschnitt | Größe | Mutationsstelle |
|---|---|---|
| I | 570 bp | - |
| II | 162 bp | 1-4 |
| III | 141 bp | 5 |
| IV | 251 bp | 6 |

| Mutationsstelle | wt-Aminosäure | erlaubte Aminosäuren |
|---|---|---|
| 1 | L | 4 (wt, K, R, M) |
| 2 | Y | 9 (wt, F, C, I, H, R, V, D, G) |
| 3 | W | 9 (wt, L, S, M, T, R, V, A, G) |
| 4 | N | 11 (wt, F, S, Y, C, I, T, V, A, D, G) |
| 5 | F | 4 (L, Q, V, E) |
| 6 | F | 6 (M, K, R, V, E, G) |

Daraus ergibt sich eine theoretische Komplexität der Bibliothek von 85536 verschiedenen Protein-Varianten.

| Mutationsstelle | Mismatch-Primer | Match-Primer |
|---|---|---|
| 1 | 3 | 1 |
| 2-4 | 972* | 1 |
| 5 | 4 | 0 |

(fortgesetzt)

| Mutationsstelle | Mismatch-Primer | Match-Primer |
|---|---|---|
| 6 | 6* | 0 |

*Hier wurden Primer-Gemische eingesetzt, die durch den Einsatz von Nukleotid-Gemischen bei der Oligonukleotid-Synthese erzeugt wurden (degenerierte Primer)

PCR-Amplifikation der Fragmente:

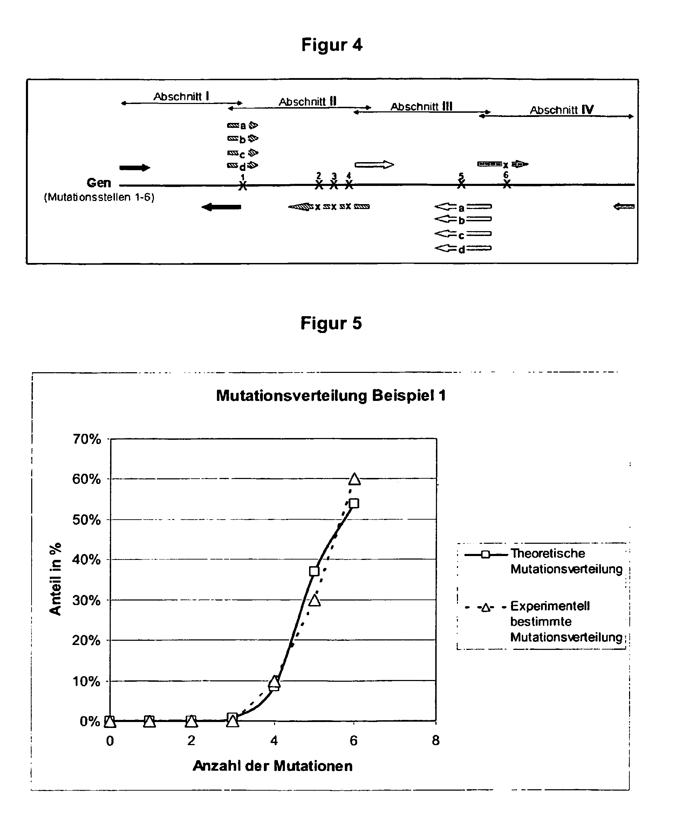

Die Sequenzabschnitte I bis IV wurden separat durch PCR amplifiziert. Gegebenenfalls wurden dabei über Mismatch-Primer gezielt Mutationen in die jeweiligen Mutationsstellen eingefügt. Figur 4 zeigt schematisch die mittels PCR amplifizierten Sequenzabschnitte. Die Pfeile symbolisieren die eingesetzen Primer. Kleine Buchstaben (a-d) stellen Mismatch-Primer mit definierten Codons dar. Die mit "x" gekennzeichneten Primer sind Mismatch-Primer die als degenerierte Primer erzeugt wurden.

Sequenzabschnitt I

Es wurde eine PCR durchgeführt, in der zwei Primer ohne Mutationsstelle eingesetzt wurden.

Sequenzabschnitt II

Es wurden vier parallele PCRs durchgeführt, indem jeweils drei Mismatch-Primer (a-c) bzw. ein Match-Primer (d) für Mutationsstelle 1 mit dem degenerierten Primer-Gemisch, welches die drei Mutationsstellen 2-4 abdeckt, kombiniert wurden.

Sequenzabschnitt III

Es wurden vier parallele PCRs durchgeführt, indem ein Primer ohne Mutationsstelle, mit vier Mismatchprimern (a-d) für kombiniert wurden.

Sequenzabschnitt IV

Es wurde eine PCR durchgeführt, indem ein das degenerierte Primer-Gemisch für Mutationsstelle 6 mit einem Primer ohne Mutationsstelle kombiniert wurde.

PCR mit der DNA-Polymerase FirePol:

|  |  |  |  |
|---|---|---|---|
|  | 10 | $\mu$l | 10xB-Puffer (Solis BioDyne |
|  | 10 | $\mu$l | 25 mM MgCl$_2$ |
|  | 2 | $\mu$l | dNTPs (je 10 mM) (Fermentas) |
|  | 1 | $\mu$l | Primer-fwd (100 mM) |
|  | 1 | $\mu$l | Primer-rev (100 mM) |
|  | 20 | ng | Plasmid mit Zielgen |
|  | 10 | U | FirePol (Solis BioDyne) |
| ad | 100 | $\mu$l | H$_2$O dest. |

94°C, 1'30" / 25 x (94°C, 20" / 55°C, 20" / 72°C, 45") / 72 °C, 10'

Die je vier PCRs für die Sequenzabschnitte II und III wurden über Agarosgel-Elektrophorese quantifiziert und jeweils äquimolar vermischt. Die Reinigung aller amplifizierten Fragmente erfolgte durch Agarosegel-Elektrophorese und Extraktion der entsprechenden Bande aus dem Gel (Gel-Reinigungskit, Promega).

Zusammenfügen der Fragmente

Das Zusammenfügen der Gene erfolgte in diesem Fall über eine Overlap-Extension-PCR. Dazu wurden die Fragmente I-IV äquimolar gemischt und mit den äußeren Primern der Sequenzabschnitt I und IV in einer PCR eingesetzt.

Overlap-Extension-PCR mit FirePol:

| | | | |
|---|---|---|---|
| | 10 | µl | 10xB-Puffer (Solis BioDyne |
| | 10 | µl | 25 mM $MgCl_2$ |
| | 2 | µl | dNTPs (je 10 mM) (Fermentas) |
| | 1 | µl | Primer-fwd (für Fragment I, 100 mM) |
| | 1 | µl | Primer-rev (für Fragment IV,100 mM) |
| | 600 | fmol | Fragment I |
| | 600 | fmol | Fragment II |
| | 600 | fmol | Fragment III |
| | 600 | fmol | Fragment IV |
| | 10 | U | FirePol (Solis BioDyne) |
| ad | 100 | µl | $H_2O$ dest. |

94°C,30" / 25 x (94°C, 20" / 60°C, 20" / 72°C, 1'10") / 72 °C, 10'

Klonierung der Sequenzen

Das entstandene Fragment wird direkt mit Restriktionsendonukleasen geschnitten und in den ebenfalls geschnittenen Expressionsvektor pRSF-1 b kloniert.

Restriktionsverdau-Ansätze:

| Fragment: | | | | Vektor: | | | |
|---|---|---|---|---|---|---|---|
| | 2 | µg | Fragment | | 5 | µg | pRSF-1 b |
| | 5 | µl | 10xO-Puffer (Fermentas) | | 7 | µl | 10xPuffer-Tango (Fermentas) |
| | 25 | U | PagI | | 30 | U | NcoI (Fermentas) |
| | 15 | U | PstI | | 20 | U | PstI (Fermentas) |
| ad | 50 | µl | $H_2O$ dest. | ad | 70 | µl | $H_2O$ dest. |

Die Restriktionsverdau-Ansätze werden 2 h bei 37°C inkubiert. Zu dem "Vektor-Ansatz" werden nach 1 h und 1,5 h zur Dephosphorylierung je 1 U SAP (Fermentas) hinzu gegeben. Anschließend werden die Enzyme für 20 min bei 80 °C inaktiviert. Daraufhin werden die gewünschten Produkte mittels Agarosegel-Elektrophorese getrennt und mit dem Gel-Reinigungs-Kit (Promega) gereinigt.
Die Vektor-DNA und das Fragment werden durch die Inkubation mit T4-DNA-Ligase wie folgt miteinander verbunden:

Ligase-Ansatz:

| | | | |
|---|---|---|---|
| | 200 | fmol | Vektor-DNA |
| | 600 | fmol | Fragment |
| | 1 | µl | 10xLigase-Puffer (Fermentas) |
| | 1 | U | T4-DNA-Ligase (Fermentas) |
| ad | 10 | µl | $H_2O$ dest. |

Die Ansätze werden 12 h bei 16 °C inkubiert und anschließend wurde die Ligase durch 10-minütiges Erhitzen auf 65 °C inaktiviert. Der Ligationsansatz wurde mittels einer Phenol-Chloroform-Extraktion gereinigt und durch Ethanolpräzipitation konzentriert. Der Ansatz wurde direkt zur Transformation von *E. coli*-Zellen XL1-Blue (Stratagene) mittels Elektroporation eingesetzt. Zur Bestimmung der erzielten Klonanzahl wurde ein Teil der elektroporierten Zellen zum Auszählen auf LB/Kanamycin-Agarplatten ausgestrichen, während der restliche Transformationsansatz in flüssigen LB/Kanamycin -Medium über Nacht bei 37 °C und 200 UpM kultiviert wurde. Ausgehend von dieser Kultur wurde die Plasmidbibliothek durch eine Midi-Plasmidpräparation mit Hilfe eines Reinigungs-Kits (Macherey+Nagel) gewonnen. Durch Auszählen der Einzelklone auf den Agarplatten wurde die Komplexität der Bibliothek auf 223000 Klone bestimmt. Damit war die theoretische Bibliotheksgröße 2,6-mal abgedeckt. Von 10 Einzelkolonien wurde das Plasmid mittels eines Plasmid-Minipräparationskits (M+N) isoliert und das enthaltene Enzym-Gen durch Sequenzierung charakterisiert.

Sequenzierung von 10 Einzelklonen der Bibliothek

| Mutationsstelle | 1 | 2 | 3 | 4 | 5 | 6 |
|---|---|---|---|---|---|---|
| **wt** | **Leu** | **Tyr** | **Trp** | **Asn** | **Phe** | **Phe** |
| Klon 1 | Met | Leu | Trp | Cys | Val | Lys |
| Klon 2 | Met | Gly | Leu | Ile | Gln | Arg |
| Klon 3 | Leu | Tyr | Gly | Ile | Val | Lys |
| Klon 4 | Lys | Phe | Val | Asn | Gln | Lys |
| Klon 5 | Met | Arg | Ala | Ile | Leu | Met |
| Klon 6 | Met | Tyr | Ala | Ser | Glu | Lys |
| Klon 7 | Arg | Phe | Val | Gly | Glu | Lys |
| Klon 8 | Lys | Phe | Leu | Cys | Glu | Arg |
| Klon 9 | Lys | Asp | Gly | Phe | Gln | Gly |
| Klon 10 | Met | Val | Met | Phe | Leu | Lys |
| **Mutationsverteilung** | 1xLeu | 2xPhe | 2xLeu | 2xPhe | 2xLeu | 1 xMet |
| | 3xLys | 2xTyr | 0xSer | 1xSer | 3xGln | 6xLys |
| | 1xArg | 1xCys | 1xTrp | 0xTyr | 2xVal | 2xArg |
| | 5xMet | 1xLeu | 1xMet | 2xCys | 3xGlu | 0xVal |
| | | 1xArg | 0xThr | 3xIle | | 0xGlu |
| | | 0xHis | 0xArg | 0xThr | | 1xGly |
| | | 1xVal | 2xVal | 1 xAsn | | |
| | | 1xAsp | 2xAla | 1 xAla | | |
| | | 1xGly | 2xGly | 0xVal | | |

0xAsp 1xGly

Er wurden also 6 Varianten mit 6 Aminosäure-Mutationen gefunden, 3 Varianten mit 5 Mutationen und 1 Variante mit 4 Mutationen. Keine Variante hatte weniger als 4 Mutationen. Die sich daraus ergebene Mutationsverteilung entspricht sehr gut der theoretisch zu erwartenden Verteilung (Figur 5)

Beispiel 2:

In diesem Beispiel ist die Erstellung einer Enzym-Varianten-Bibliothek mit Hilfe des erfindungsgemäßen Verfahrens beschrieben. Die Ausgangssequenz ist das Gen der Lipase B aus *Candida antarctica* (CalB). Innerhalb der Gen-Sequenz wurden 5 zu mutierende Aminosäuren (K32, Q58, R242, R249, E269) ausgewählt, die auf insgesamt 5 Sequenzabschnitten liegen.

| Sequenzabschnitt | Größe | Mutationsstelle (Aminosäure) |
|---|---|---|
| I | 167 bp | K32 |
| II | 99 bp | Q58 |
| III | 570 bp | R242 |

(fortgesetzt)

| Sequenzabschnitt | Größe | Mutationsstelle (Aminosäure) |
|---|---|---|
| IV | 78 bp | R249 |
| V | 396 bp | E269 |

| wt-Aminosäure | erlaubte Aminosäuren |
|---|---|
| K32 | 11 (wt, P, L, F, W, S, Y, E, Q, G, A) |
| Q58 | 11 (wt, P, L, F, W, S, Y, E, R, G, A) |
| R242 | 11 (wt, P, L, F, W, S, Y, E, Q, G, A) |
| R249 | 11 (wt, P, L, F, W, S, Y, E, Q, G, A) |
| E269 | 11 (wt, P, L, F, W, S, Y, R, Q, G, A) |

Daraus ergibt sich eine theoretische Komplexität der Bibliothek von 161.051 verschiedenen Protein-Varianten.

PCR-Amplifikation der Fragmente:

Die Sequenzabschnitte I bis V wurden separat durch PCR amplifiziert. Gegebenenfalls wurden dabei über Mismatch-Primer gezielt Mutationen in die jeweiligen Mutationsstellen eingefügt. Für jeden der fünf Sequenzabschnitte wurden 11 parallele PCRs durchgeführt, indem jeweils 10 Mismatch-Primer bzw. ein Match-Primer (wt) für die Mutationsstelle mit einem Primer ohne Mutationsstelle kombiniert wurden.

Sequenzabschnitte I bis V

PCR mit der DNA-Polymerase Phusion™ Hot Start:

| | | | |
|---|---|---|---|
| | 20 | µl | 5xHF-Puffer (Finnzymes) |
| | 2 | µl | dNTPs (je 10 mM) (Fermentas) |
| | 5 | µl | Primer-fwd (10 mM) |
| | 5 | µl | Primer-rev (10 mM) |
| | 20 | ng | Plasmid-DNA mit Zielgen |
| | 2 | U | Phusion™ Hot Start (Finnzymes) |
| ad | 100 | µl | $H_2O$ dest. |

98°C, 30" / 30 x (98°C, 10" / 65°C, 20" / 72°C, 10") / 72 °C, 10'

Die je 11 PCRs für die Sequenzabschnitte I bis V wurden über Agarosgel-Elektrophorese quantifiziert und jeweils äquimolar vermischt. Die Reinigung aller amplifizierten Fragmente erfolgte durch Agarosegel-Elektrophorese und Extraktion der entsprechenden Bande aus dem Gel (Gel-Reinigungskit, Promega).

Zusammenfügen der Fragmente

Das Zusammenfügen der Gene erfolgte im Beispiel über eine Overlap-Extension-PCR. Dazu wurden die Fragmente I-V äquimolar gemischt und mit den äußeren Primern der Sequenzabschnitt I und V in einer PCR eingesetzt.

Overlap-Extension-PCR mit *PfuUltra*™ HF-DNA-Polymerase:

| | | |
|---|---|---|
| 10 | µl | 10x *PfuUltra*™ HF Puffer (Stratagene) |
| 2 | µl | dNTPs (je 10 mM) (Fermentas) |
| 2,5 | µl | Primer I-fwd (10 mM) |
| 2,5 | µl | Primer V-rev (10 mM) |

(fortgesetzt)

| | 600 | fmol | Fragment I |
|---|---|---|---|
| | 600 | fmol | Fragment II |
| | 600 | fmol | Fragment III |
| | 600 | fmol | Fragment IV |
| | 600 | fmol | Fragment V |
| | 2,5 | U | *PfuUltra*™ (Stratagene) |
| ad | 100 | µl | H$_2$O dest. |

95°C,2' / 20 x (95°C, 30" / 54°C, 30" / 72°C, 1'30") / 72 °C, 10'

Klonierung der Sequenzen

Das entstandene Fragment wird direkt mit Restriktionsendonukleasen geschnitten und in den ebenfalls geschnittenen Expressionsvektor pEXP kloniert.

Restriktionsverdau-Ansätze:

| Fragment: | | | | Vektor: | | | |
|---|---|---|---|---|---|---|---|
| | 5 | µg | Fragment | | 20 | µg | pEXP |
| | 20 | µl | 10xR-Puffer (Fermentas) | | 40 | µl | 10xR-Puffer (Fermentas) |
| | 30 | U | XhoI (Fermentas) | | 240 | U | XhoI (Fermentas) |
| | 20 | U | NotI (Fermentas) | | 100 | U | NotI (Fermentas) |
| ad | 200 | µl | H$_2$O dest. | ad | 400 | µl | H$_2$O dest. |

Die Restriktionsverdau-Ansätze werden 12 h bei 37°C inkubiert. Zu dem "Vektor-Ansatz" werden nach 11 h und 11,5 h zur Dephosphorylierung je 4 U CIAP (Fermentas) hinzu gegeben. Anschließend werden die Enzyme durch eine Phenol-Chloroform-Extraktion inaktiviert. Daraufhin werden die gewünschten Produkte mittels Agarosegel-Elektropgorese getrennt und mit dem Gel-Reinigungs-Kit (Promega) gereinigt.

Die Vektor-DNA und das Fragment werden durch die Inkubation mit T4-DNA-Ligase wie folgt miteinander verbunden:

Ligase-Ansatz:

| | 500 | fmol | Vektor-DNA |
|---|---|---|---|
| | 1500 | fmol | Fragment |
| | 6 | µl | 10xLigase-Puffer (Fermentas) |
| | 15 | U | T4-DNA-Ligase (Fermentas) |
| ad | 60 | µl | H$_2$O dest. |

Die Ansätze werden 10 h bei 16 °C inkubiert und anschließend wurde die Ligase durch 15-minütiges Erhitzen auf 65 °C inaktiviert. Der Ligationsansatz wurde mittels einer Phenol-Chloroform-Extraktion gereinigt und durch Ethanolpräzipitation konzentriert. Der Ansatz wurde direkt zur Transformation von *E. coli*-Zellen XL1-Blue (Stratagene) mittels Elektroporation eingesetzt. Zur Bestimmung der erzielten Klonanzahl wurde ein Teil der elektroporierten Zellen zum Auszählen auf Selektions-LB-Agarplatten ausgestrichen, während der restliche Transformationsansatz in flüssigen Selektions-LB-Medium über Nacht bei 30 °C und 200 UpM kultiviert wurde. Ausgehend von dieser Kultur wurde die Plasmidbibliothek durch eine Midi-Plasmidpräparation mit Hilfe eines Reinigungs-Kits (Macherey+Nagel) gewonnen. Durch Auszählen der Einzelklone auf den Agarplatten wurde die Komplexität der Bibliothek auf 1,33 Mio Klone bestimmt. Damit war die theoretische Bibliotheksgröße 8,3-mal abgedeckt. Von 10 Einzelkolonien wurde das Plasmid mittels eines Plasmid-Minipräparationskits (M+N) isoliert und das enthaltene Enzym-Gen durch Sequenzierung charakterisiert.

Sequenzierung von 10 Einzelklonen der Bibliothek

| Mutationsstelle | K32 | Q58 | R242 | R249 | E269 |
|---|---|---|---|---|---|
| **CalB wt** | **K** | **Q** | **R** | **R** | **E** |
| Klon 1 | | A | S | P | W |
| Klon 2 | Q | A | G | W | W |
| Klon 3 | Q | S | Y | Y | S |
| Klon 4 | S | S | G | S | Y |
| Klon 5 | S | R | Y | W | W |
| Klon 6 | Q | E | S | F | A |
| Klon 7 | E | S | Y | R | F |
| Klon 8 | E | Q | W | F | W |
| Klon 9 | E | P | L | Q | E |
| Klon 10 | W | W | W | R | P |
| **Mutationsverteilung** | 0xK | 1xQ | 0xR | 2xR | 1xE |
| | 0xP | 1xP | 0xP | 1xP | 1xP |
| | 0xL | 0xL | 1xL | 0xL | 0xL |
| | 0xF | 0xF | 0xF | 2xF | 1xF |
| | 1 xW | 1xW | 2xW | 2xW | 4xW |
| | 2xS | 3xS | 2xS | 1xS | 1xS |
| | 0xY | 0xY | 3xY | 1xY | 1xY |
| | 3xE | 1xE | 0xE | 0xE | 0xR |
| | 3xQ | 1xR | 0xQ | 1xQ | 0xQ |
| | 1 xG | 0xG | 2xG | 0xG | 0xG |
| | 0xA | 2xA | 0xA | 0xA | 1xA |

Er wurden also 6 Varianten mit 5 Aminosäure-Mutationen und 4 Varianten mit 4 Mutationen Keine Variante hatte weniger als 4 Mutationen.

**Patentansprüche**

1.   Verfahren zur Erzeugung einer Varianten-Bibliothek von DNA-Sequenzen ausgehend von mindestens einer DNA-Ausgangssequenz umfassend die folgenden Schritte:

a) Auswählen von mindestens zwei Mutationsstellen in der DNA-Ausgangssequenz;
b) Unterteilen der DNA-Ausgangssequenz in mindestens zwei Sequenzabschnitte in einer Weise, so dass mindestens zwei dieser Sequenzabschnitte jeweils mindestens eine der Mutationsstellen umfassen;
c) Amplifizieren der Sequenzabschnitte durch PCR mit Hilfe von insgesamt wenigstens fünf verschiedenen Oligonukleotiden, wobei

(i) sich an jede der Mutationsstellen mindestens eines der Oligonukleotide anlagern kann;
(ii) sich an mindestens eine Mutationsstelle wenigstens zwei der Oligonukleotide anlagern können; und
(iii) in den durch PCR erhaltenen doppelsträngigen Amplifikations-Produkten durch die Oligonukleotide an den Mutationsstellen über *Mismatch*-Positionen Mutationen eingeführt werden, wobei an mindestens einer der Mutationsstellen mindestens zwei Mutationen eingeführt werden; und

d) Verknüpfen der doppelsträngigen Amplifikations-Produkte zu DNA-Sequenzen;
wobei

(i) für mindestens 75% der Varianten in der Varianten-Bibliothek die Anzahl der Mutationen pro Variante im Bereich N liegt, wobei $X \geq N \geq (X-3)$ mit $X \in N$ und $25 > X > 3$, oder
(ii) die größte Fraktion der Varianten eine Anzahl d von Mutationen aufweist und die Fraktion der Varianten, welche d+2 Mutationen aufweist, höchstens 10% der Gesamtzahl der Varianten in der Varianten-Bibliothek ausmacht.

**2.** Verfahren nach Anspruch 1, **dadurch gekennzeichnet, dass** in Schritt c) die Amplifikation der Sequenzabschnitte jeweils räumlich getrennt voneinander erfolgt.

**3.** Verfahren nach Anspruch 1 oder 2, **dadurch gekennzeichnet, dass** in Schritt c) die Amplifikation von mindestens 2 Varianten mindestens eines Sequenzabschnitts jeweils räumlich getrennt voneinander erfolgt.

**4.** Verfahren nach Anspruch 2 oder 3, **dadurch gekennzeichnet, dass** die räumlich voneinander getrennten Amplifikations-Produkte vermischt werden, bevor sie in Schritt d) verknüpft werden.

**5.** Verfahren nach Anspruch 4, wobei die Amplifikations-Produkte vor dem Mischen quantifiziert werden.

**6.** Verfahren nach einem der vorstehenden Ansprüche, **dadurch gekennzeichnet, dass** die Oligonukleotide unterteilt werden können in

(a) *Mismatch*-Oligonukleotide, welche jeweils an mindestens eine Mutationsstelle anlagern können und dort bei der Amplifikation durch PCR über *Mismatch*-Positionen Mutationen einführen, und
(b) *Match*-Oligonukleotide, welche jeweils an mindestens einen Sequenzabschnitt anlagern können und bei der Amplifikation durch PCR an keiner Mutationsstelle eine Mutation einführen,

wobei die Anzahl der *Mismatch*-Oligonukleotide kleiner, gleich oder größer als die Anzahl der *Match*-Oligonukleotide ist.

**7.** Verfahren nach einem der vorstehenden Ansprüche, **dadurch gekennzeichnet, dass** die DNA-Ausgangssequenz ein Gemisch von homologen Sequenzen umfasst.

**8.** Verfahren nach einem der vorstehenden Ansprüche, **dadurch gekennzeichnet, dass** in der erzeugten Varianten-Bibliothek die Anzahl der Varianten, die genauso viele Mutationen wie Mutationsstellen aufweisen, größer ist als die Anzahl der Varianten, deren Anzahl von Mutationen um 1 kleiner ist als die Anzahl der Mutationsstellen.

**9.** Verfahren nach einem der vorstehenden Ansprüche, **dadurch gekennzeichnet, dass** in der Varianten-Bibliothek weniger als 1,0% DNA-Ausgangssequenzen enthalten sind.

**10.** Verfahren nach einem der vorstehenden Ansprüche, **dadurch gekennzeichnet, dass** für mindestens 75% der Varianten in der Varianten-Bibliothek die Anzahl der Mutationen pro Variante im Bereich N liegt, wobei $X \geq N \geq (X-2)$ mit $X \in N$ und $20 > X > 2$.

**11.** Verfahren nach einem der vorstehenden Ansprüche, **dadurch gekennzeichnet, dass** für mindestens 75% der Varianten in der Varianten-Bibliothek die Anzahl der Mutationen pro Variante im Bereich N liegt, wobei $X \geq N \geq (X-1)$ mit $X \in N$ und $20 > X > 1$.

## Claims

**1.** Method for generating a variant library of DNA sequences starting from at least one DNA starting sequence, comprising the following steps:

a) selecting at least two mutation sites in the DNA starting sequence;
b) dividing the DNA starting sequence into at least two sequence segments in such a way that at least two of these sequence segments each comprise at least one of the mutation sites;
c) amplifying the sequence segments by PCR with the aid of at least five different oligonucleotides in total, wherein

(i) at least one of the oligonucleotides can attach to each of the mutation sites;
(ii) at least two of the oligonucleotides can attach to at least one mutation site; and
(iii) in the double-stranded amplification products obtained by PCR, mutations are introduced by the oligonucleotides at the mutation sites via mismatch positions, wherein at at least one of the mutation sites, at least two mutations are introduced; and

d) linking the double-stranded amplification products to form DNA sequences;
wherein

(i) for at least 75% of the variants in the variant library, the number of mutations per variant is in the range N, where $X \geq N \geq (X-3)$ with $X \varepsilon N$ and $25 > X > 3$, or
(ii) the largest fraction of the variants has a number d of mutations and the fraction of the variants which has d+2 mutations makes up not more than 10% of the total number of variants in the variant library.

2. Method according to Claim 1, **characterized in that**, in step c), the sequence segments are amplified in each case in a spatially separated manner.

3. Method according to Claim 1 or 2, **characterized in that**, in step c), at least 2 variants of at least one sequence segment are amplified in each case in a spatially separated manner.

4. Method according to Claim 2 or 3, **characterized in that** the spatially separated amplification products are mixed before they are linked in step d.

5. Method according to Claim 4, wherein the amplification products are quantified before mixing.

6. Method according to any of the preceding claims, **characterized in that** the oligonucleotides can be divided into

(a) mismatch oligonucleotides, each of which can attach to at least one mutation site and which introduce mutations there via mismatch positions during amplification by PCR, and
(b) match oligonucleotides, each of which can attach to at least one sequence segment and which do not introduce any mutation at any mutation site during amplification by PCR,

wherein the number of mismatch oligonucleotides is smaller, the same, or greater than the number of match oligonucleotides.

7. Method according to any of the preceding claims, **characterized in that** the DNA starting sequence comprises a mixture of homologous sequences.

8. Method according to any of the preceding claims, **characterized in that**, in the variant library generated, the number of variants which have exactly the same number of mutations and mutation sites is greater than the number of variants whose number of mutations is less by 1 than the number of mutation sites.

9. Method according to any of the preceding claims, **characterized in that** the variant library contains less than 1.0% DNA starting sequences.

10. Method according to any of the preceding claims, **characterized in that**, for at least 75% of the variants in the variant library, the number of mutations per variant is in the range N, where $X \geq N \geq (X-2)$ with $X \varepsilon N$ and $20 > X > 2$.

11. Method according to any of the preceding claims, **characterized in that**, for at least 75% of the variants in the variant library, the number of mutations per variant is in the range N, where $X \geq N \geq (X-1)$ with $X \varepsilon N$ and $20 > X > 1$.

**Revendications**

1. Procédé de production d'une banque de variants de séquences d'ADN à partir d'au moins une séquence d'ADN de départ, comprenant les étapes suivantes :

a) sélection d'au moins deux sites de mutation dans la séquence d'ADN de départ ;
b) subdivision de la séquence d'ADN de départ en au moins deux segments de séquence, de telle sorte qu'au moins deux de ces segments de séquence comprennent chacun au moins l'un des sites de mutation ;
c) amplification des segments de séquence par PCR à l'aide d'en tout au moins cinq oligonucléotides différents,

(i) au moins l'un des oligonucléotides pouvant se fixer à chacun des sites de mutation ;

(ii) au moins deux des oligonucléotides pouvant se fixer à au moins un site de mutation ; et

(iii) des mutations étant introduites par l'intermédiaire des oligonucléotides au niveau des sites de mutation, sur des positions de mésappariement, dans les produits d'amplification double brin obtenus par PCR ; et

d) liaison des produits d'amplification double brin à des séquences d'ADN ;
dans lequel

(i) pour au moins 75 % des variants de la banque de variants, le nombre de mutations par variant est compris dans la plage N, avec $X \geq N \geq (X-3)$, avec $X \in N$ et $25 > X > 3$, ou

(ii) la fraction la plus grande des variants comprend un nombre d de mutations, et la fraction des variants qui présente d+2 mutations représente plus 10 % du nombre total des variants dans la banque des variants.

2. Procédé selon la revendication 1, **caractérisé en ce que**, dans l'étape c), l'amplification de chacun des segments de séquence s'effectue spatialement séparément les uns des autres.

3. Procédé selon la revendication 1 ou 2, **caractérisé en ce que**, dans l'étape c), l'amplification d'au moins 2 variants d'au moins un segment de séquence s'effectue spatialement séparément les uns des autres.

4. Procédé selon la revendication 2 ou 3, **caractérisé en ce que** les produits d'amplification spatialement séparés les uns des autres sont mélangés avant d'être reliés dans l'étape d).

5. Procédé selon la revendication 4, dans lequel les produits d'amplification sont quantifiés avant le mélange.

6. Procédé selon l'une des revendications précédentes, **caractérisé en ce que** les oligonucléotides peuvent être subdivisés en

(a) des oligonucléotides mésappariés, dont chacun peut se fixer à au moins un site de mutation, et y introduire des mutations, lors de l'amplification par PCR, sur les positions de mésappariement, et

(b) des oligonucléotides appariés, dont chacun peut se fixer à au moins un segment de séquence et, lors de l'amplification par PCR, n'introduit de mutation sur aucun site de mutation,

dans lequel le nombre des oligonucléotides mésappariés est inférieur, égal ou supérieur au nombre des oligonucléotides appariés.

7. Procédé selon l'une des revendications précédentes, **caractérisé en ce que** la séquence d'ADN de départ comprend un mélange de séquences homologues.

8. Procédé selon l'une des revendications précédentes, **caractérisé en ce que**, dans la banque de variants produite, le nombre des variants qui présentent exactement le même nombre de mutations que de sites de mutation est supérieur au nombre des variants dont le nombre de mutations est inférieur de 1 au nombre des sites de mutations.

9. Procédé selon l'une des revendications précédentes, **caractérisé en ce que** la banque de variants contient moins de 1,0 % de séquences d'ADN de départ.

10. Procédé selon l'une des revendications précédentes, **caractérisé en ce que**, pour au moins 75 % des variants de la banque des variants, le nombre des mutations par variant est compris dans la plage N, avec $X \geq N \geq (X-2)$, avec $X \in N$ et $20 > X > 2$.

11. Procédé selon l'une des revendications précédentes, **caractérisé en ce que**, pour au moins 75 % des variants de la banque de variants, le nombre des mutations par variant est compris dans la plage N, avec $X \geq N \geq (X-1)$, et $X \in N$ et $20 > X > 1$.

# Figur 1

## Figur 2

## Figur 3

**Mutationsverteilung**

## Figur 4

## Figur 5

Mutationsverteilung Beispiel 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 01112802 A
- WO 0234762 A
- WO 0112802 A
- WO 03106679 A
- WO 9832845 A
- WO 9858080 A
- WO 2006047669 A


**In der Beschreibung aufgeführte Nicht-Patentliteratur**

- **L. GE et al.** *Biotechniques,* 1997, vol. 22 (1), 28-30
- **E. P. WEISBERG et al.** *Biotechniques,* 1993, vol. 15 (1), 68-76
- **R. M. SHAYIQ et al.** *Analytical Biochemistry,* 1994, vol. 221 (1), 206-208
- **W. ITO et al.** *Gene,* 1991, vol. 102 (1), 67-70
- **WONG T. S. et al.** The Diversity Challenge in Directed Protein Revolution. *Combinatorial Chemistry & High Throughput Screening,* 2006, vol. 9 (4), 271-288
- **C. NEYLON.** *Nucleic Acids Research,* 2004, vol. 32 (4), 1448-59
- **J OSUNA et al.** *Gene,* 1991, vol. 106 (1), 7-12
- **S. BRAKMANN et al.** Directed Molecular Evolution of Proteins. VCH-Wiley, 2002
- **F.H. ARNOLD.** Directed Enzyme Evolution: Screening and Selection Methods. Humana Press, 2003
- **C.W. DIEFFENBACH et al.** PCR Primer: A Laboratory Manual. Cold Spring Harbor Laboratory Press, 2003
- **M.J. MCPHERSON et al.** PCR (The Basics. Taylor & Francis, 2006
- **M. ALTSHULER.** PCR Troubleshooting: The Essential Guide. Caister Academic Press, 2006